(19) Europäisches Patentamt
European Patent Office
Office européen des brevets

(11) EP 2 861 556 B1

(12) # FASCICULE DE BREVET EUROPEEN

(45) Date de publication et mention de la délivrance du brevet:
**12.07.2017 Bulletin 2017/28**

(21) Numéro de dépôt: **13739801.2**

(22) Date de dépôt: **04.06.2013**

(51) Int Cl.:
***C07C 211/55*** (2006.01) ***C07C 209/28*** (2006.01)
***C07C 211/14*** (2006.01) ***C07D 295/04*** (2006.01)
***C07B 43/04*** (2006.01) ***C07C 211/08*** (2006.01)
***C07C 211/48*** (2006.01) ***C07D 295/02*** (2006.01)

(86) Numéro de dépôt international:
**PCT/IB2013/054599**

(87) Numéro de publication internationale:
**WO 2013/182991 (12.12.2013 Gazette 2013/50)**

(54) **PROCÉDÉ DE PRÉPARATION D'AMINES MÉTHYLÉES**

VERFAHREN ZUR HERSTELLUNG VON METHYLIERTEN AMINEN

METHOD FOR PREPARING METHYLATED AMINES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.06.2012 FR 1255234**

(43) Date de publication de la demande:
**22.04.2015 Bulletin 2015/17**

(73) Titulaire: **Commissariat à l'énergie atomique et aux énergies alternatives**
**75015 Paris (FR)**

(72) Inventeurs:
- **CANTAT, Thibault**
  **F-92130 Issy Les Moulineaux (FR)**
- **GOMES, Christophe**
  **F-92160 Antony (FR)**
- **JACQUET, Olivier**
  **F-91400 Orsay (FR)**

(74) Mandataire: **Gevers & Orès**
**41 avenue de Friedland**
**75008 Paris (FR)**

(56) Documents cités:
- **SALVATORE R N ET AL: "Efficient Cs2CO3-promoted solution and solid phase synthesis of carbonates and carbamates in the presence of TBAI", TETRAHEDRON, ELSEVIER SCIENCE PUBLISHERS, AMSTERDAM, NL, vol. 58, no. 17, 22 avril 2002 (2002-04-22), pages 3329-3347, XP004349219, ISSN: 0040-4020, DOI: 10.1016/S0040-4020(02)00286-7 cité dans la demande**
- **GREDIG S V ET AL: "PALLADIUM CATALYZED SYNTHESIS OF METHYLAMINES FROM CARBON DIOXIDE, HYDROGEN AND AMMONIA", CATALYSIS LETTERS, SPRINGER NEW YORK LLC, UNITED STATES, vol. 46, no. 1/02, 1 juin 1997 (1997-06-01), pages 49-55, XP000692742, ISSN: 1011-372X, DOI: 10.1023/A:1019085511301**
- **S. RAM ET AL.: "Rapid reductive-carboxylation of secondary amines. One pot synthesis of tertiary N-methylated amines.", TETRAHEDRON LETTERS, vol. 26, no. 44, 1 janvier 1985 (1985-01-01), pages 5367-5370, XP002690975, cité dans la demande**
- **S. RAM ET AL.: "REGIO- AND CHEMOSELECTIVE N-C BOND FORMATION VIA CARBON DIOXIDE: A NEW SOURCE OF THE METHYL GROUP", SYNTHETIC COMMUNICATIONS, vol. 19, no. 20, 1 janvier 1989 (1989-01-01), pages 3561-3571, XP002690976, cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- S.T.RIDUAN ET AL.: "conversion of carbon dioxide into methanol with silanes over N-heterocyclic carbene catalysts", ANGEWANDTE CHEMIE INTERNATIONAL EDITION, vol. 48, 1 janvier 2009 (2009-01-01), pages 3322-3325, XP002690977, cité dans la demande
- O. JACQUET ET AL.: "CO2 as a C1 buiding block for the catalytic methylation of amines", CHEMICAL SCIENCE, vol. 4, 24 February 2013 (2013-02-24), pages 2127-2131,
- L. GONZALÈS-SEBASTIÁN ET AL.: "Selective N-methylation of aliphatic amines with CO2 and hydrosilanes using nickel-phosphine catalysts", ORGANOMETALLICS, vol. 34, 30 January 2015 (2015-01-30), pages 763-769,
- SHOUBHIK DAS ET AL.: "Metal-free catalyst for the chemoselctive methylation of amines using carbon dioxide as a carbon source", ANGEWANDTE COMMUNICATIONS, vol. 53, 26 September 2014 (2014-09-26), pages 12876-12879,
- F. LIGER ET AL.,: "Direct [11C]methylation of amines from [11C]CO2 for the synthesis of PET radiotracers", EUR. J. ORG. CHEM., 10 July 2015 (2015-07-10),

## Description

La présente invention concerne un procédé de préparation d'amines méthylées utilisant le dioxyde de carbone et l'utilisation de ce procédé dans la fabrication de vitamines, de produits pharmaceutiques, de colles, de fibres acryliques et cuirs synthétiques, de pesticides, et d'engrais.

Elle concerne également un procédé de fabrication de vitamines, de produits pharmaceutiques, de colles, de fibres acryliques, de cuirs synthétiques, de pesticides, et d'engrais, comprenant une étape de préparation d'amines méthylées par le procédé selon l'invention.

La présente invention concerne, en outre, un procédé de préparation d'amines méthylées marquées et leurs utilisations.

L'utilisation du $CO_2$ valorisable comme source de carbone pour la production de consommables chimiques est un défi de premier plan pour diminuer son accumulation atmosphérique mais également pour contrôler notre dépendance en combustibles fossiles.

Le plus grand défi auquel font face les scientifiques et industriels est de recycler le $CO_2$, c'est-à-dire de développer des réactions permettant de produire des composés chimiques comme, par exemple, des combustibles, des polymères plastiques, des médicaments, des détergents, des molécules à hauts tonnages, traditionnellement obtenus par des méthodes pétrochimiques. La difficulté technique réside dans la mise au point de réactions chimiques qui permettent de fonctionnaliser le $CO_2$ tout en réduisant le centre carboné (i.e. en substituant les liaisons C-O du $CO_2$ par des liaisons C-H ou C-C).

Compte tenu de la grande stabilité thermodynamique du dioxyde de carbone, sa conversion vers de nouveaux consommables chimiques fait nécessairement appel à une source énergétique extérieure de manière à favoriser le bilan thermodynamique de la transformation chimique représenté en Figure 1.

Aujourd'hui, l'ensemble des efforts de la communauté scientifique se focalise sur l'emploi d'électricité ou de lumière pour réaliser l'électroréduction ou la photoréduction du $CO_2$ en acide formique, méthanal, méthanol et méthane (Morris, A. J., Meyer, G. J., Fujita, E., Accounts Chem Res 2009, 42, 1983). De fait, ce domaine de recherche fait l'objet d'une compétition internationale intense.

Un article récent décrit que l'utilisation de composés silanes permet de réduire le $CO_2$ en conditions organocatalytiques (Riduan, S. N., Zhang, Y. G., Ying, J. Y., Angewandte Chemie-International Edition 2009, 48, 3322). Dans ce cas, le composé silane est l'espèce réactive haute en énergie, et l'utilisation du catalyseur favorise le bilan cinétique. Les auteurs décrivent la formation de produits silylés de types formyl (SiOCHO), acétal ($SiOCH_2OSi$) et méthoxy ($SiOCH_3$). Si cette stratégie est justifiée par l'importance des utilisations des produits de réduction du $CO_2$ dans l'industrie chimique (HCOOH, $H_2CO$, $CH_3OH$), il convient néanmoins de noter que ces molécules sont actuellement utilisées sur une échelle qui reste très faible par rapport à la quantité de $CO_2$ valorisable disponible. En d'autres termes, si ces molécules étaient produites exclusivement à partir de $CO_2$, elles ne permettraient de valoriser, compte tenu du marché actuel, que 3,4% du $CO_2$ valorisable produit chaque année (2,5 Gt/an) (Panorama des voies de valorisation du $CO_2$, ADEME, Juin 2010, http://www2.ademe.fr/servlet/getDoc?cid=96&m=3&id=72052&p1=:30&ref=12441). Ainsi, il est nécessaire de chercher à diversifier la nature et le nombre de consommables chimiques que l'on peut obtenir à partir du $CO_2$.

Une autre stratégie de conversion du $CO_2$ vers de nouveaux consommables chimiques, consiste à utiliser un partenaire chimique réactif (haut en énergie) pour favoriser le bilan thermodynamique de la transformation chimique de $CO_2$. Cette stratégie est encore très peu représentée dans le paysage scientifique mais elle permettra, à terme, d'ouvrir considérablement l'offre de molécules disponibles à partir de $CO_2$. Le seul procédé industriel reposant sur cette approche est la synthèse de l'urée obtenue par condensation d'ammoniac sur $CO_2$ comme indiqué dans l'équation 1 ci-dessous (Sakakura, T., Choi, J. C., Yasuda, H., Chem Rev 2007, 107, 2365).

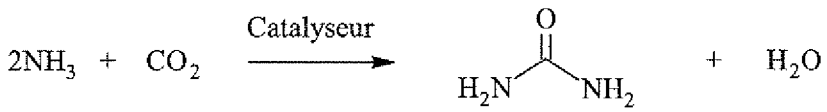


(équation 1)

Selon le même principe, la synthèse de polycarbonates par copolymérisation $CO_2$/époxydes est en voie d'industrialisation comme indiqué dans l'équation 2 ci-dessous (Panorama des voies de valorisation du $CO_2$, ADEME, Juin 2010, http://www2.ademe.fr/servlet/getDoc?cid=96&m=3&id=72052&p1=30&ref=12441).

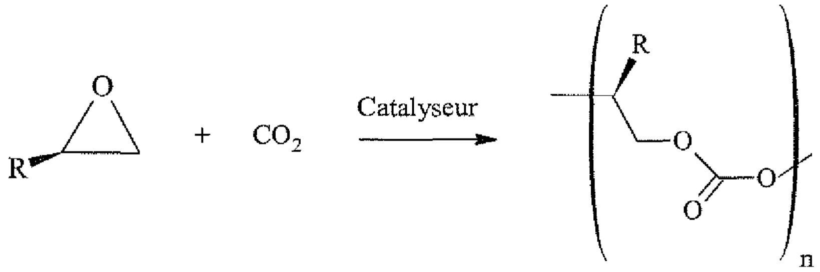


(équation 2)

Dans ces deux synthèses (équations 1 et 2), il n'y a aucune réduction formelle du centre carboné de $CO_2$.

Toujours dans le but d'obtenir de nouveaux consommables chimiques, on peut envisager de convertir le $CO_2$ en composés aminés, et plus spécifiquement en amines méthylés. Les amines méthylées (de formule générale $R^1R^2NCH_3$) sont une classe de composés chimiques importants dans l'industrie chimique où ils sont couramment utilisés comme solvants, réactifs, engrais, herbicides, fongicides, principes actifs pour les médicaments, et précurseurs de matériaux plastiques (Amines: Synthesis, Properties and Applications, Lawrence, S. A., Cambridge University Press, 2006 ; Arpe, H.-J.; Hawkins, S. Industrial Organic Chemistry; Wiley-VCH: Weinheim , 1997 ; Ali, M. F.; El Ali, B. M.; Speight, J. G. Handbook of Industrial Chemistry - Organic Chemicals; McGraw-Hill: New York, 2005.).

Les amines méthylées (de formule générale $R^1R^2NCH_3$) sont généralement synthétisées par réaction entre une amine de formule générale $R^1R^2NH$ et d'un agent de méthylation électrophile tel que l'iodure de méthyle, le méthanol, le diméthylsulfate ou le diméthylcarbonate, préférentiellement en présence d'une base.

Alternativement, les amines méthylées peuvent être obtenues en employant du paraformaldéhyde, en présence d'un réducteur ($H_2$, $NaBH_4$).

Ces différentes voies de synthèse ne mettent donc pas en jeu le $CO_2$ comme source de carbone pour la méthylation de la liaison N-H de l'amine.

La synthèse d'amines méthylées à partir du $CO_2$ est très peu décrite. Elle est, notamment, décrite par trois publications :

- En 1985, Ram et Ehrenkaufer ont décrit la carboxylation d'amines en présence de $CO_2$. Après alkylation ou silylation, les esters carbamiques obtenus sont réduits par du tétrahydruroaluminate de lithium ($LiAlH_4$) (S. Ram, R. E. Ehrenkaufer, Tetrahedron Lett., 1985, Vol. 26, Issue 44, p. 5367-5370).
- Suivant une stratégie similaire, une méthode en trois étapes a été mise au point par Jung et al. : la première étape consiste à réaliser la carbonatation de l'amine en présence de carbonate de césium. Dans une deuxième étape, le carbamate ainsi formé est greffé de façon covalente sur une résine dite de Merrifield. Enfin, par réduction au tétrahydruroaluminate de lithium ($LiAlH_4$), l'ester carbamique supporté sur résine est réduit en amine méthylée (R. N. Salvatore, F. X. Chu, A. S. Nagle, E. A. Kapxhiu, R. M. Cross, K. W. Jung, Tetrahedron, 2002, Vol. 58, p. 3329-3347).
- Une stratégie différente a été développée par Ram et Spicer en 1989. Elle repose sur la silylation de la liaison N-H d'une amine par l'hexaméthyldisilazane ($Me_3SiSiMe_3$) puis réaction avec du $CO_2$, en présence de tétrahydruroaluminate de lithium ($LiAlH_4$) (S. Ram, L. D. Spicer, Synthetic Communications 1989, Vol. 19, p. 3561-3571).

Ces voies de synthèse présentent des inconvénients, en particulier :

- la source d'hydrures est du $LiAlH_4$, un réducteur dur incompatible avec la présence de groupements fonctionnels sur les amines ;
- les procédés font intervenir plusieurs étapes qui nécessitent des purifications intermédiaires ;
- les réactions ne sont pas catalytiques ce qui impose l'utilisation de réactifs puissants (tels que $LiAlH_4$) et le recours à des étapes multiples pour améliorer les rendements et sélectivités.

Dans le cadre de la synthèse des amines méthylées en utilisant le dioxyde de carbone, le défi technique à relever est de coupler la fonctionnalisation du dioxyde de carbone (formation d'une liaison C-N) à une étape de réduction chimique (formation de trois liaisons C-H). Pour maximiser le rendement énergétique d'une telle transformation, il est nécessaire de développer des réactions avec un nombre limité d'étapes (idéalement une seule) et catalysées, pour éviter les pertes énergétiques d'ordre cinétique.

Par ailleurs, les amines méthylées marquées, incorporant des radioisotopes et/ou isotopes stables, présente un intérêt particulier dans de nombreux domaines comme, par exemple, dans les sciences du vivant (étude/élucidation de mécanismes enzymatiques, de mécanismes biosynthétiques, en biochimie, etc.), les sciences de l'environnement (traçage de déchets, etc.), la recherche (étude/élucidation de mécanismes réactionnels) ou encore la recherche et le

développement de nouveaux produits pharmaceutiques et thérapeutiques. Ainsi, développer une synthèse pour la préparation d'amines méthylées marquées répondant aux exigences indiquées ci-dessus, répond à un besoin réel.

Il existe donc un réel besoin d'un procédé pour préparer les amines méthylées, par transformation du $CO_2$, palliant les inconvénients de l'art antérieur, ledit procédé permettant de coupler la fonctionnalisation du dioxyde de carbone à une étape de réduction chimique.

En particulier, il existe un réel besoin d'un procédé qui permette d'obtenir en une seule étape et avec une bonne voire excellente sélectivité les amines méthylées, à partir du $CO_2$ et d'amines, en conditions catalytiques et en présence d'un composé assurant la réduction de $CO_2$ et qui soit compatible avec la présence de groupements fonctionnels sur l'amine.

Il existe, en outre, un réel besoin de disposer d'un procédé qui permette d'obtenir, en une seule étape et avec une excellente sélectivité, les amines méthylées marquées incorporant des radioisotopes et/ou isotopes stables, à partir de réactifs marqués comme par exemple le $CO_2$ marqué et/ou les amines marquées, en conditions catalytiques et en présence d'un composé assurant la réduction de $CO_2$ et qui soit compatible avec la présence de groupements fonctionnels sur l'amine.

La présente invention a précisément pour but de répondre à ces besoins en fournissant un procédé de préparation d'amines méthylées de formule (I) :

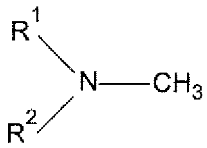

(I)

dans laquelle

- $R^1$ et $R^2$, représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, un groupe amino, une aldimine de formule $-N{=}CHR^6$, une cétimine de formule $-N{=}CR^6R^7$, lesdits groupes alkyle, alcényle, alcynyle, aryle, hétéroaryle, hétérocycle, silylé, siloxy et amino étant éventuellement substitués, ou
- $R^1$ et $R^2$, pris ensemble avec l'atome d'azote auquel ils sont liés forment un hétérocycle éventuellement substitué, ou
- $R^1$ et $R^2$, forment avec l'atome d'azote auquel ils sont liés, une double liaison carbone-azote (N=C) pour conduire à une aldimine de formule $-N{=}CHR^6$ ou à une cétimine de formule $-N{=}CR^6R^7$,

  et
- $R^6$ et $R^7$, représentant, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, un groupe amino, lesdits groupes alkyle, alcényle, alcynyle, aryle, hétéroaryle, hétérocycle, silylé, siloxy et amino étant éventuellement substitués,
- $R^1$, $R^2$, $R^6$ et $R^7$ comportent éventuellement, un H, C, N, O, F, Si et/ou S tels que définis ci-dessous ;
- H représente un atome d'hydrogène ($^1H$), le deutérium ($^2H$) ou le tritium ($^3H$);
- C représente un atome de carbone ($^{12}C$), un isotope $^{11}C$, $^{13}C$ ou $^{14}C$ ;
- N représente un atome d'azote ($^{14}N$), un isotope $^{15}N$ ;
- O représente un atome d'azote ($^{16}O$), un isotope $^{18}O$ ;
- F représente un atome d'azote ($^{19}F$), un isotope $^{18}F$ ;
- Si représente un atome de silicium ($^{28}Si$), un isotope $^{29}Si$ ou $^{30}Si$ ;
- S représente un atome de soufre ($^{32}S$), un isotope $^{33}S$, $^{34}S$ ou $^{36}S$ ;

caractérisé en ce que l'on fait réagir une amine de formule (II) dans laquelle $R^1$ et $R^2$ et N sont tels que définis ci-dessus,

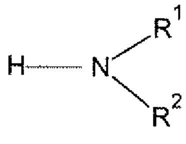

(II)

avec du $CO_2$ dans lequel C et O sont tels que définis ci-dessus ; en présence d'un catalyseur et d'un composé silane de formule (III)

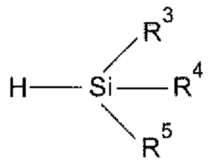

(III)

dans laquelle

- H est tel que défini ci-dessus,
- $R^3$, $R^4$ et $R^5$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^5$ est tel que défini ci-dessus et $R^3$ et $R^4$, pris ensemble avec l'atome de silicium auquel ils sont liés forment un hétérocycle silylé éventuellement substitué.

Le procédé de l'invention a l'avantage de permettre de convertir le $CO_2$ éventuellement marqué, en amines méthylées éventuellement marquées avec un grand choix d'amines de formule (II) (amines primaires, secondaires, aromatiques, aliphatiques, etc.) éventuellement marquées. Dans ce procédé, lesdites amines servent essentiellement à fonctionnaliser le $CO_2$, et les composés silanes de formule (III) assurent la réduction de $CO_2$, en conditions catalytiques.

Les amines méthylées sont ainsi obtenues avec un bon rendement (de l'ordre de 35 à 100%, par exemple), et une bonne voire excellente sélectivité (par exemple, plus de 50% voire plus de 70% d'amines méthylées isolées).

Dans le cadre de la présente invention, le rendement est calculé par rapport à la quantité d'amine de formule (II) introduite initialement, sur la base de la quantité d'amine méthylée isolée :

$$\text{Rendement} = \text{n(amine méthylée)}/(\text{n(amine méthylée)}+\text{n(amine)})$$

n étant la quantité de matière

Dans le cadre de la présente invention, la sélectivité se rapporte à la nature des produits formés à partir de l'amine de formule (II).

Comme déjà indiqué, le procédé de l'invention permet d'obtenir les amines méthylées de formule (I) en « une seule étape ». Autrement dit, contrairement aux procédés de l'état de la technique dans lesquels les amines de départ sont soumises a des réactions chimiques successives avec ajout successif (un à la fois) des autres réactifs (comme les bases, les composés silylés, les réducteurs notamment le $LiAlH_4$, etc.), et avec ou sans séparation des produits intermédiaires, le procédé de l'invention se fait en « une seule étape » au cours de laquelle l'ensemble des réactifs se trouvent simultanément dans le milieu réactionnel. Ainsi, sur le plan industriel le procédé de l'invention présente un grand intérêt car il permet de gagner en temps, en coût de production et en rendement global.

On entend par « alkyle », au sens de la présente invention, un radical carboné linéaire, ramifié ou cyclique, saturé ou insaturé, éventuellement substitué, comprenant 1 à 12 atomes de carbone. A titre d'alkyle saturé, linéaire ou ramifié, on peut citer par exemple les radicaux méthyle, éthyle, propyle, butyle, pentyle, hexyle, heptyle, octyle, nonyle, décyle, undécyle, dodécanyle et leurs isomères ramifiés. Comme alkyle cylique, on peut citer les radicaux cyclopropyle, cyclobutyle, cyclopentyle, cyclohexyle, bicylco[2,1,1] hexyle, bicyclo[2,2,1] heptyle. Comme alkyles cycliques insaturés, on peut citer par exemple le cyclopentényle, le cyclohexényle. Les alkyles insaturés appelés également « alcényle » ou « alcynyle » contiennent respectivement au moins une double ou une triple liaison. A ce titre, on peut citer, par exemple, les radicaux éthylényle, propylényle, butényle, pentényle, hexényle, acétylényle, propynyle, butynyle, pentynyle, hexynyle et leurs isomères ramifiés. Le groupe alkyle, au sens de l'invention incluant les groupes alcényle et alcynyle, peut être éventuellement substitué par un ou plusieurs groupes hydroxyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle, avec les groupes alkoxy et aryle tels que définis dans le cadre de la présente invention.

Le terme « aryle » désigne de manière générale un substituant aromatique cyclique comportant de 6 à 20 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un

ou plusieurs groupes alkyle, un ou plusieurs groupes aryle, avec les groupes alkoxy, alkyle et aryle tels que définis dans le cadre de la présente invention.

Le terme « hétéroaryle » désigne de manière générale un substituant aromatique mono- ou polycyclique comportant de 5 à 10 membres dont au moins 2 atomes de carbone, et au moins un hétéroatome choisi parmi l'azote, l'oxygène ou le soufre. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, triazolyle, pyrazolyle, oxazolyle, isoxazolyle, benzoxazolyle, thiazolyle, benzothiazolyle, isothiazolyle, pyridazinyle, pyrimidilyle, pyrimidinyle, pyrazinyle, triazinyle, cinnolinyle, phtalazinyle, quinazolinyle, 1,1-diphénylhydrazinyle, 1,2-diphénylhydrazinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes aryle, un ou plusieurs groupes alkyle, avec les groupes alkyle, alkoxy et aryle tels que définis dans le cadre de la présente invention.

Le terme « alkoxy » signifie un groupe alkyle, tel que défini ci-dessus, lié par un atome d'oxygène (-O-alkyle).

Le terme « hétérocycle » désigne de manière générale un substituant mono- ou polycyclique, comportant de 5 à 10 membres, saturé ou instauré, contenant de 1 à 4 hétéroatomes choisis indépendamment l'un de l'autre, parmi l'azote, l'oxygène et le soufre. A titre indicatif, on peut citer les substituants morpholinyle, pipéridinyle, pipérazinyle, pyrrolidinyle, imidazolyle, imidazolidinyle, imidazolinyle, pyrazolidinyle, tétrahydrofuranyle, tétrahydropyranyle, tétrahydropyrimidinyle, triazolyle, pyrazolyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Par atome d'halogène, on entend un atome choisi parmi les atomes de fluor, chlore, brome ou iode.

Par groupe « silylé », on entend un groupe de formule $[-Si(X)_{3]}$ dans lequel chaque X, indépendamment l'un de l'autre, est choisi parmi un atome d'hydrogène ; un ou plusieurs atomes d'halogène choisis parmi les atomes de fluor, chlore, brome ou iode ; un ou plusieurs groupes alkyle ; un ou plusieurs groupes alkoxy ; un ou plusieurs groupes aryle ; un ou plusieurs groupes siloxy ; avec les groupes alkyle, alkoxy, aryle et siloxy tels que définis dans le cadre de la présente invention. Lorsque l'un au moins des X représente plusieurs groupes siloxy, lesdits groupes siloxy peuvent se répéter plusieurs fois de manière à conduire à des organosilanes polymériques de formule générale

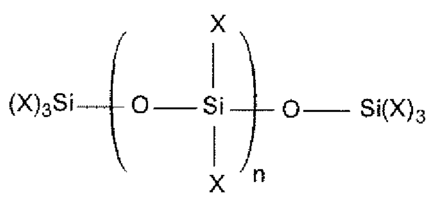

dans laquelle X est tel que défini ci-dessus et n est un nombre entier compris entre 1 et 20000, avantageusement entre 1000 et 5000. A ce titre on peut citer, par exemple, le polydiméthyl siloxane (PDMS) ou le polyméthylhydroxysiloxane (PMHS).

Par groupe « siloxy », on entend un groupe silylé, tel que défini ci-dessus, lié par un atome d'oxygène ($-O-Si(X)_3$).

Au sens de l'invention, par « hétérocycle silylé », on entend un substituant mono-ou polycyclique, comportant de 5 à 15 membres, saturé ou insaturé, contenant au moins un atome de silicium et éventuellement au moins un autre hétéroatome choisi parmi l'azote, l'oxygène ou le soufre. Ledit hétérocycle silylé peut être éventuellement substitué par un ou plusieurs groupes hydroxyle ; un ou plusieurs groupes alkyle, un ou plusieurs groupes alkoxy ; un ou plusieurs atomes d'halogène choisis parmi les atomes de fluor, chlore, brome et iode ; un ou plusieurs groupes aryle, avec les groupes alkyle, alkoxy et aryle tels que définis dans le cadre de la présente invention. Parmi les hétérocycles silylés, on peut citer par exemple, le 1-silacyclo-3-pentène ou le 1-méthyl-1,1-dihydrido-2,3,4,5-tétraphényl-1-silacyclopentadiène, suivant les formules ci-dessous.

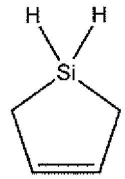

1-silacyclo-3-pentène

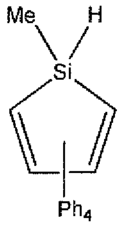

1-méthyl-1-hydrido-2,3,4,5-tétraphényl-1-silacyclopentadiène

On peut encore citer, par exemple, le méthyl siloxane, le 1-phényl-1-silacyclohexane, le 1-sila-bicyclo[2.2.1]heptane, le 1-méthyl-1-silacyclopentane, le 9,9-dihydro-5-silafluorène répondant aux formules ci-dessous.

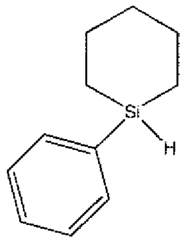

1-phenyl-1-silacyclohexane

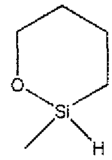

methyl siloxane

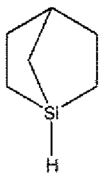

1-sila-bicyclo[2.2.1]heptane

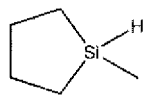

1-methyl-1-silacyclopentane

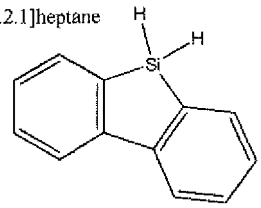

9,9-dihydro-5-silafluorene

Les hétérocycles silylés de l'invention peuvent être disponibles commercialement ou peuvent, le cas échéant, être préparés par les procédés de synthèse connus comme, par exemple, décrits par C.L. Smith et al., Journal of Organometallic Chemistry, 81 (1974), p. 33-40 ; G.D. Homer, Journal of the American Chemical Society, 95:23, (1973), p. 7700-7707 ; L. Spialter et al., Journal of the American Chemical Society, 93:22 (1971), p. 5682-5686 ; R. West, Journal of the American Chemical Society (1954), p. 6015-6017. L'homme du métier sera en mesure de mettre en oeuvre et d'adapter les procédés connus à la synthèse des différents hétérocycles silylés.

Par groupe « amino », on entend un groupe de formule $-NR^6R^7$, dans laquelle : $R^6$ et $R^7$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, siloxy, tels que définis dans le cadre de la présente invention ; ou $R^6$ et $R^7$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

Lorsque $R^1$ et $R^2$, forment avec l'atome d'azote auquel ils sont liés, une double liaison carbone-azote (N=C) pour conduire à une aldimine de formule $-N=CHR^6$ ou à une cétimine de formule $-N=CR^6R^7$, et que $R^6$ et $R^7$, représentent, indépendamment l'un de l'autre, un groupe alkyle, un groupe alcényle, un groupe alcynyle, un groupe aryle, un groupe hétéroaryle, un hétérocycle, un groupe silylé, un groupe siloxy, un groupe amino, lesdits groupes alkyle, alcényle,

alcynyle, aryle, hétéroaryle, hétérocycle, silylé, siloxy et amino tels que définis dans le cadre de la présente invention, peuvent être éventuellement substitués par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Les substituants, radicaux et groupes définis ci-dessus peuvent comporter, éventuellement, du deutérium ($^2H$), du tritium ($^3H$), du $^{11}C$, du $^{13}C$, du $^{14}C$, du $^{15}N$, du $^{18}O$, du $^{18}F$, du $^{29}Si$, du $^{30}Si$, du $^{33}S$, du $^{34}S$ ou du $^{36}S$.

Lorsque les composés de formule (I), (II) et (III) comportent au moins un radiomarqueur/radiotraceur ou un isotope, ils peuvent également être désignés par les formules (I'), (II') et (III').

Selon une variante préférée de l'invention, dans l'amine de formule (II), $R^1$ et $R^2$, représentent, indépendamment l'un de l'autre, un atome d'hydrogène, un groupe alkyle, un groupe aryle, un groupe hétéroaryle, lesdits groupes alkyle, aryle et hétéroaryle étant éventuellement substitués, ou

$R^1$ et $R^2$, pris ensemble avec l'atome d'azote auquel ils sont liés forment un hétérocycle éventuellement substitué.

De préférence, dans l'amine de formule (II), $R^1$ et $R^2$, représentent, indépendamment l'un de l'autre, un atome d'hydrogène ; un groupe alkyle choisi parmi les groupes méthyle, éthyle, propyle, butyle, pentyle, hexyle, heptyle, ou leurs isomères ramifiés, cyclohexyle ; un groupe aryle choisi parmi le benzyle ou le phényle ; un groupe hétéroaryle choisi parmi l'imidazolyle ou le benzimidazolyle ; ou

$R^1$ et $R^2$, pris ensemble avec l'atome d'azote auquel ils sont liés forment un hétérocycle comportant de 5 à 6 membres choisi parmi la morpholine ; la pipéridine ; la pipérazine ; la pyrrolidine ; l'oxazolidine ; ou l'isoxazolidine ; l'imidazole, en particulier, le 1H-imidazole; la tétrahydropyrimidine, en particulier, la 1,4,5,6-tétrahydropyrimidine ; triazolyle, pyrazolyle.

Selon une autre variante préférée de l'invention, dans le composé silane de formule (III), $R^3$, $R^4$ et $R^5$ représentent, indépendamment l'un de l'autre, un atome d'hydrogène, un groupe alkyle, un groupe alkoxy, un groupe aryle, un groupe silylé, un groupe siloxy, lesdits groupes alkyle, alkoxy, silylé, siloxy et aryle étant éventuellement substitués.

De préférence, dans le composé silane de formule (III), $R^3$, $R^4$ et $R^5$ représentent indépendamment l'un de l'autre,

- un atome d'hydrogène ;
- un groupe alkyle choisi parmi les groupes méthyle, éthyle, propyle, butyle, pentyle, hexyle, heptyle ou leurs isomères ramifiés ;
- un groupe alkoxy dont le groupe alkyle est choisi parmi les groupes méthyle, éthyle, propyle, butyle, pentyle, hexyle, heptyle ou leurs isomères ramifiés ;
- un groupe aryle choisi parmi les groupes benzyle ou phényle ;
- un groupe siloxy ;
- un groupe silylé de formule $[-Si(X)_3]$ dans lequel chaque X, indépendamment l'un de l'autre, est choisi parmi un atome d'hydrogène, un ou plusieurs atomes d'halogène choisis parmi les atomes chlore, brome, ou iode, un ou plusieurs groupes alkyle choisi parmi les groupes méthyle, éthyle, propyle, butyle, pentyle, hexyle, heptyle ou leurs isomères ramifiés, un ou plusieurs groupes alkoxy dont le groupe alkyle est choisi parmi les groupes méthyle, éthyle, propyle, butyle, pentyle, hexyle, heptyle ou leurs isomères ramifiés, un ou plusieurs groupes siloxy dont le groupe $-Si(X)_3$ est tel que décrit dans ce mode de réalisation ; plusieurs groupes siloxy se répétant plusieurs fois conduisant à des organosilanes polymériques de formule générale

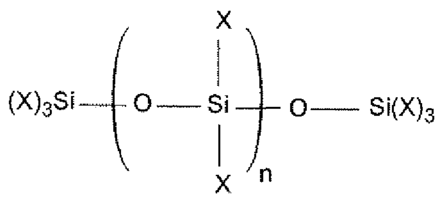

dans laquelle X est tel que défini dans ce mode de réalisation et n est un nombre entier compris entre 1 et 20000, avantageusement entre 1000 et 5000.

Par catalyseur, au sens de l'invention, on entend tout composé capable de modifier, notamment en augmentant, la vitesse de la réaction chimique à laquelle il participe, et qui est régénéré à la fin de la réaction. Cette définition englobe à la fois les catalyseurs, c'est-à-dire les composés qui exercent leur activité catalytique sans avoir besoin de subir une quelconque modification ou conversion, et les composés (appelés également pré-catalyseurs) qui sont introduits dans le milieu réactionnel et qui y sont convertis en un catalyseur.

Les catalyseurs peuvent être choisis parmi les catalyseurs organiques ou les catalyseurs métalliques, les

catalyseurs métalliques étant choisis parmi les sels ou les complexes métalliques. Les catalyseurs organiques présentent l'avantage de permettre de s'affranchir des problèmes de toxicité généralement observés pour les catalyseurs métalliques ainsi que des problèmes de coûts associés à l'utilisation de métaux précieux. Dans le procédé de l'invention, le catalyseur est, de préférence, organique.

Les catalyseurs organiques sont, en général, des bases organiques, choisies parmi :

- les bases azotées, comme par exemple, les amines secondaires ou tertiaires choisies parmi le triazabicyclodécène (TBD) ; le N-méthyltriazabicyclodécène (MeTBD), le 1,8-diazabicyclo[5.4.0]undéc-7-ène (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ;
- les bases phosphorées, comme par exemple, les alkyles et aryle phosphines choisis parmi le triphénylphosphine, le 2,2'-bis(diphénylphosphino)-1,1'-binaphthyle (BINAP), le triisopropylphosphine ; les alkyle et aryle phosphonates choisis parmi le diphénylphosphate, le triphénylphosphate (TPP), le tri(isopropylphényl)phosphate (TIPP), le crésyldiphenyl phosphate (CDP), le tricrésylphosphate (TCP) ; les alkyle et aryle phosphates choisis parmi le di-n-butylphosphate (DBP), le tris-(2-éthylhexyl)-phosphate, le triéthyl phosphate ;
- les bases carbonées pour lesquelles la protonation a lieu sur un atome de carbone, comme par exemple, un carbène N-hétérocyclique comme un carbène issu d'un sel d'imidazolium choisi parmi les sels de 1,3-bis(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant, par exemple, sous la forme de sels de chlorure comme représentés ci-dessous :

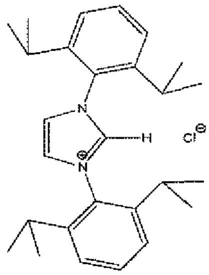
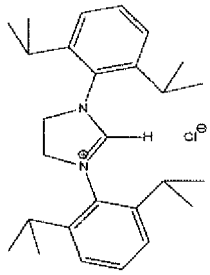
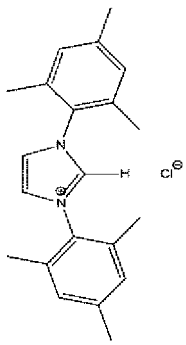
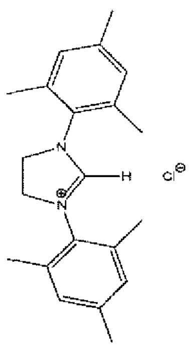

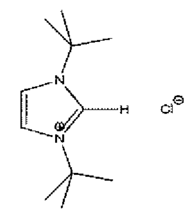
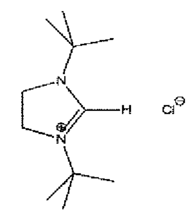
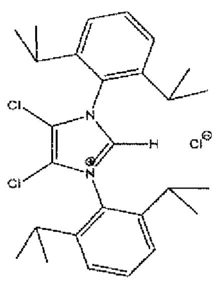

ou

- les bases oxygénées, comme par exemple le peroxyde d'hydrogène ; le peroxyde de benzoyle ; un alcoolate choisi parmi le méthanolate, l'éthanolate, le propanolate, le butanolate, le pentanolate, l'hexanolate, de sodium ou de potassium.

Le catalyseur organique est avantageusement :

- une amine secondaire ou tertiaire choisie parmi le triazabicyclodécène (TBD) ; le N-méthyltriazabicyclodécène (MeTBD), le 1,8-diazabicyclo[5.4.0]undéc-7-ène (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ; ou
- un carbène N-hétérocyclique comme un carbène issu d'un sel d'imidazolium choisi parmi les sels de 1,3-bis(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant, par exemple, sous la forme de sels de chlorure comme représentés ci-dessous :

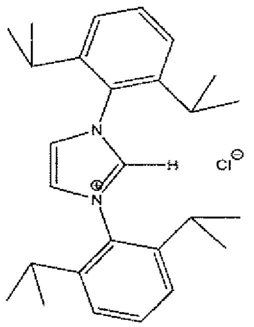
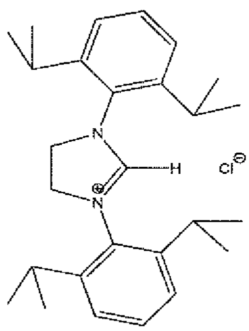
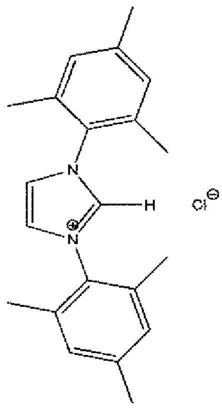
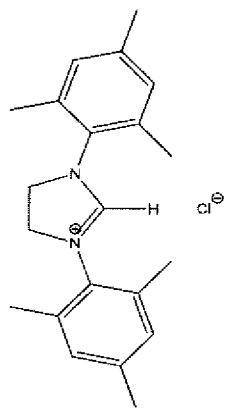

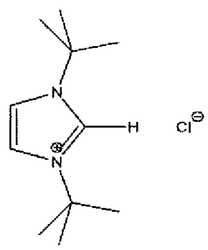
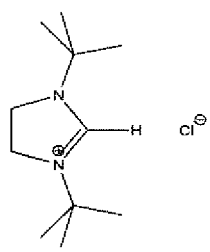
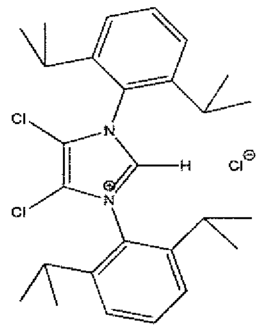

Selon une variante préférée de l'invention, le catalyseur organique est choisi parmi le triazabicyclodécène (TBD), le N-méthyltriazabicyclodécène (MeTBD), le 1,8-diazabicyclo[5.4.0]undéc-7-ène (DBU) ; ou un carbène N-hétérocyclique tel qu'un carbène issu d'un sel d'imidazolium tel que le chlorure de 1,3-bis(2,6-diisopropylphenyl)-1H-imidazol-3-ium (carbène A), le chlorure de 1,3-bis(2,6-diisopropylphenyl)-4,5-dihydro-1H-imidazol-3-ium (carbène C), le chlorure de 1,3-di-tert-butyl-1H-imidazol-3-ium, le chlorure de 1,3-di-tert-butyl-4,5-dihydro-1H-imidazol-3-ium (carbène F), le chlorure de 1,3-bis(2,4,6-triméthylphenyl)-1H-imidazol-3-ium (carbène B), le chlorure de 1,3-bis(2,4,6-triméthylphenyl)-4,5-dihydro-1H-imidazol-3-ium (carbène D), le chlorure de 4,5-dichloro-1,3-bis(2,6-diisopropylphenyl)-1H-imidazol-3-ium (carbène E).

Lorsque le catalyseur est un catalyseur métallique, il peut être choisi parmi les sels ou complexes de :

- métaux choisis parmi le bore, le silicium, l'aluminium, le gallium, l'étain, l'indium ;
- métaux alcalins choisis parmi le sodium, le potassium ;
- métaux alcalinoterreux choisis le magnésium, le calcium ;
- métaux de transition choisis parmi le nickel, le fer, le cobalt, le zinc, le cuivre, le rhodium, le ruthénium, le platine, le palladium, l'iridium ;
- terres rares choisis parmi le lanthane, le cérium, le praséodyme, le néodyme.

A titre d'exemples, le catalyseur métallique, peut être choisi parmi les sels ou complexes suivants :

- $Al(OiPr)_3$, $SnCl_2$, $InBr_3$ en tant que sels ou complexes de métaux ;
- $Na_2CO_3$, $K_2CO_3$, $Cs_2CO_3$ en tant que sels ou complexes de métaux alcalins ;
- $MgSO_4$, $Ca(BH_4)_2$ en tant que sels ou complexes de métaux alcalinoterreux ;
- $Fe(BH_4)_2.6H_2O$, $Fe(BF_4)_2.6H_2O$, $Fe(acac)_3$, CuCl, $Cu(OAc)_2(H_2O)$, $Zn(OAc)_2$, Zn(BDI)Et, $ZnEt_2$, $ZnCl_2$, $ZnSO_4$, en tant que sels ou complexes de métaux de transition ;
- $La(OTf)_3$, $CeCl_3$ en tant que sels ou complexes de terres-rares.

Par complexe métallique il est entendu un composé organométallique ou inorganique de coordination dans lequel un ion métallique est lié à un ligand organique ou inorganique. Un complexe organométallique ou inorganique peut être obtenu par mélange d'un sel métallique avec un ligand, celui-ci se liant au métal par des atomes de phosphore, de carbone, d'azote, d'oxygène, d'hydrogène ou de silicium, par exemple. Comme ligand organique ou inorganique, et à titre indicatif on peut citer un ligand du type phosphine ou amine comme, par exemple, le tris[2-diphénylephosphino)éthyle]phosphine ($PP_3$), le carbène A, la tricyclohexylphosphine, l'acétate (AcO), l'acétylacétonate (acac), le 1,2-bis-diphénylphosphinoéthane (dppe), le *N,N,N',N'*-tétra-méthyl-éthylènediamine (TMEDA), le *N,N'*-bis(2,6-diisopropylphenyl) β-dicétiminate (BDI), le 1,2- bis(diphénylphosphino) éthane (dppb), ou la pyridine.

Selon une variante préférée de l'invention, le catalyseur métallique est obtenu :

- par mélange d'un sel métallique de fer comme par exemple $Fe(acac)_3$, $Fe(acac)_2$, ou $Fe(BF_4)_2(H_2O)_6$, avec un ligand de type phosphine ou amine comme par exemple TMEDA, dppe, $PP_3$ ; ou bien
- par mélange d'un sel de zinc comme par exemple Zn(BDI)Et, $Zn(OAc)_2$, ou $ZnEt_2$ avec un ligand de type amine comme par exemple TMEDA, pyridine, le carbène A.

Certaines des abréviations utilisées pour les ligands sont représentées ci-dessous :

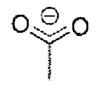
ligand OAc

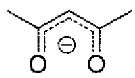
ligand acac

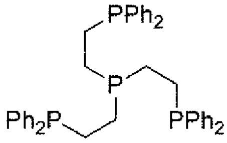
$PP_3$

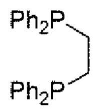
dppe

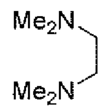
TMEDA

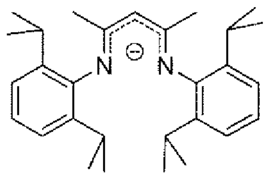
ligand BDI

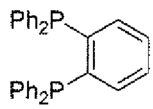
dppb

Sans vouloir être lié par la théorie, l'amine $R^1R^2NH$ sert à fonctionnaliser le $CO_2$ par la formation d'intermédiaires de type carbamates et le silane assure l'étape de réduction desdits intermédiaires de type carbamates.

Les catalyseurs peuvent, le cas échéant, être immobilisés sur des supports hétérogènes afin d'assurer une séparation facile dudit catalyseur et/ou son recyclage. Lesdits supports hétérogènes peuvent être choisis parmi les supports à base de gel de silice ou de polymères plastiques comme, par exemple, le polystyrène ; les supports carbonés choisis parmi les nanotubes de carbone ; le carbure de silice ; l'alumine ; ou le chlorure de magnésium ($MgCl_2$).

Dans le procédé selon l'invention, la réaction peut se produire sous une pression de $CO_2$, en faisant barboter du $CO_2$ dans le milieu réactionnel ou sous une atmosphère sèche contenant du $CO_2$ (air ambiant séché comprenant, par exemple environ 78% en volume d'azote, 21% en volume d'oxygène, et d'environ de 0,2 à 0,04% en volume de dioxyde de carbone). La réaction peut également se produire en utilisant du $CO_2$ supercritique.

De préférence, la réaction se produit sous une pression de $CO_2$.

La pression du $CO_2$, peut alors être comprise entre 1 et 50 bars, de préférence entre 1 et 30, plus préférentiellement entre 1 et 10 bars, bornes incluses.

La température de la réaction peut être comprise entre 25 et 150°C, de préférence entre 50 et 125°C, plus préférentiellement entre 70 et 100°C, bornes incluses.

La durée de la réaction dépend du taux de conversion de l'amine de formule (II). La réaction est avantageusement maintenue jusqu'à la conversion totale de l'amine de formule (II). La réaction peut être effectuée pendant une durée de 5 minutes à 72 heures, avantageusement de 15 minutes à 48 heures, de préférence de 1 à 48 heures, bornes incluses.

Le procédé de l'invention, en particulier la réaction entre les différents réactifs, peut avoir lieu dans un ou un mélange d'au moins deux solvant(s) choisi(s) parmi :

- les éthers, de préférence, l'éther diéthylique, ou le THF ;
- les hydrocarbures, de préférence, le benzène, ou le toluène ;
- les solvants azotés, de préférence, la pyridine, ou l'acétonitrile ;
- les sulfoxydes, de préférence, le diméthylesulfoxyde ;
- les halogénures d'alkyle, de préférence, le chloroforme, ou le chlorure de méthylène.

Selon une variante préférée de l'invention, il n'est pas nécessaire d'ajouter un solvant supplémentaire. Dans ce cas l'amine de formule (II) est le solvant. Ainsi, outre son rôle de fonctionnalisation du $CO_2$, l'amine sert de solvant.
Le rapport molaire entre le composé silane de formule (III) et l'amine de formule (II) est de 1 à 10, de préférence de 1 à 3, bornes incluses.
La quantité de catalyseur est de 0,001 à 1 équivalent molaire, de préférence de 0,001 à 0,9 équivalent molaire, plus préférentiellement de 0,01 à 0,9 équivalent molaire, encore plus préférentiellement de 0,01 à 0,5 équivalent molaire, bornes incluses, par rapport à l'amine de formule (II).
Les différents réactifs utilisés dans le procédé de l'invention(les amines de formule (II), les composés silane de formule (III), les (pré-)catalyseurs etc.) sont, en général, des composés commerciaux ou peuvent être préparés par tout procédé connu de l'homme du métier.
L'invention concerne également, le procédé de préparation d'amines méthylées marquées de formule (I') :

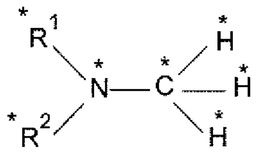

(I')

dans laquelle

- $R^{1*}$, $R^{2*}$, $R^{6*}$ et $R^{7*}$ sont tels que définis précédemment, et comportent éventuellement un $H^*$, $C^*$, $N^*$, $O^*$, $F^*$, $Si^*$ et/ou $S^*$ tels que définis ci-dessous ;
- $H^*$ représente un atome d'hydrogène ($^1H$), le deutérium ($^2H$) ou le tritium ($^3H$) ;
- $C^*$ représente un atome de carbone ($^{12}C$), un isotope $^{11}C$, $^{13}C$ ou $^{14}C$ ;
- $N^*$ représente un atome d'azote ($^{14}N$), un isotope $^{15}N$ ;
- $O^*$ représente un atome d'azote ($^{16}O$), un isotope $^{18}O$ ;
- $F^*$ représente un atome d'azote ($^{19}F$), un isotope $^{18}F$ ;
- $Si^*$ représente un atome de silicium ($^{28}Si$), un isotope $^{29}Si$ ou $^{30}Si$ ;
- $S^*$ représente un atome de soufre ($^{32}S$), un isotope $^{33}S$, $^{34}S$ ou $^{36}S$ ;

caractérisé en ce que l'on fait réagir une amine de formule (II') dans laquelle $R^{1*}$, $R^{2*}$ et $N^*$ sont tels que définis ci-dessus,

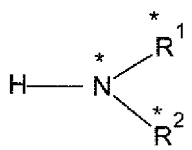

(II')

avec du $C^*O^*_2$ dans lequel $C^*$ et $O^*$ sont tels que définis ci-dessus ; en présence d'un catalyseur et d'un composé silane de formule (III')

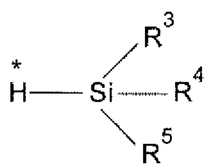

(III')

dans laquelle

- $R^3$, $R^4$, $R^5$ et $H^*$ sont tels que définis ci-dessus.

Les composés de formule (I') correspondent en fait aux composés de formule (I) comportant au moins un radiomarqueur/radiotraceur ou un isotope choisi.
Par isotopes on entend, pour un même élément, deux atomes ayant le même nombre de protons (et d'électrons) mais un nombre de neutrons différent. Ayant le même nombre d'électrons et de protons, les propriétés chimiques des

isotopes d'un même élément sont presque identiques. Il peut exister, cependant, de légères variations de la vitesse d'une réaction chimique lorsqu'un des atomes d'un réactif est remplacé par un de ses isotopes. En revanche, comme le noyau ne comporte pas le même nombre de neutrons, la masse des atomes varie ce qui peut rendre l'atome instable : c'est pourquoi ils peuvent être radioactifs. Il s'agit alors de radioisotopes. Dans le cadre de l'invention, le terme « isotopes » peut également englober les « radioisotopes ».

Le radiomarquage est le fait d'associer à une molécule ou un composé donnés un isotope qui permettra de suivre l'évolution ou/et la fixation des molécules, par exemple, dans un organe. Le radiotraceur est le ou le(s) élément(s) radioactif(s) présent(s) au sein d'une molécule pour suivre le cheminement de cette substance, par exemple, dans un organe.

Ce procédé peut ainsi permettre l'accès aux amines méthylées marquées $^{11}C$, $^{13}C$, $^{14}C$, $^{15}N$, $^{2}H$ (D) et/ou $^{3}H$ (T).

L'utilisation de molécules à des fins de traçage, de métabolisation, d'imagerie, etc., est détaillée dans la littérature (U. Pleiss, R. Voges, "Synthesis and Applications of Isotopically Labelled Compounds, Volume 7", Wiley-VCH, 2001 ; R. Voges, J. R. Heys, T. Moenius, "Preparation of Compounds Labeled with Tritium and Carbon-14". Wiley-VCH: Chippenham (UK), 2009). La possibilité de former les amines méthylées marquées peut être assurée par la disponibilité des réactifs marqués correspondant, par exemple, par :

- les amines $R^1R^2NH$ enrichies en $^{15}N$ sont accessibles à partir du chlorure d'ammonium enrichi $^{15}N$ : $[^{15}NH_4][Cl]$ (Yong-Joo Kim, Max P. Bernstein, Angela S. Galiano Roth, Floyd E. Romesberg, Paul G. Williard, David J. Fuller, Aidan T. Harrison, and David B. Collum, J. Org. Chem. 1991, 56, p. 4435-4439) ;
- les amines $R^1R^2NH$ avec $R^1$ et/ou $R^2$ marqués sont préparées par les voies de synthèses détaillées par U. Pleiss, R. Voges, "Synthesis and Applications of Isotopically Labelled Compounds, Volume 7", Wiley-VCH, 2001 ; et R. Voges, J. R. Heys, T. Moenius, "Preparation of Compounds Labeled with Tritium and Carbon-14", Wiley-VCH: Chippenham (UK), 2009) ;
- le $CO_2$ marqué $^{11}C$ ou $^{14}C$ est la source principale de $^{11}C$ et $^{14}C$ est obtenue par acidification du carbonate de baryum marqué $Ba^{14}CO_3$. (R. Voges, J. R. Heys, T. Moenius, "Préparation of Compounds Labeled with Tritium and Carbon-14". Wiley-VCH: Chippenham (UK), 2009) ;
- les silanes $R^3R^4R^5Si$-H marqués $^{2}H$ (deutérium ou D) ou $^{3}H$ (tritium ou T) sont accessibles à partir du chlorosilane correspondant $R^3R^4R^5Si$-Cl et d'hydrure de lithium (LiH) ou tétrahydruroaluminate de lithium ($LiAlH_4$), les hydrures étant disponibles tous deux en versions deutérée et tritiée (T. A. Kochina, D. V. Vrazhnov, E. N. Sinotova, V. V. Avrorin, M. Yu. Katsap, and Yu. V. Mykhov, Russian Journal of General Chemistry, Vol. 72, No. 8, 2002, p. 1222-1224 ; E. A. Shishigin, V. V. Avrorin, T. A. Kochina, and E. N. Sinotova, Russian Journal of General Chemistry, Vol. 75, No. 1, 2005, p. 152).

De préférence le $CO_2$ marqué $^{11}C$ ou $^{14}C$ est utilisé dans le procédé de préparation d'amines méthylées marqués de formule (I').

Les molécules marquées $^{14}C$ ont contribué à de nombreuses avancées dans les sciences du vivant (mécanismes enzymatiques, mécanismes biosynthétiques, biochimie), les sciences de l'environnement (traçage de déchets), la recherche (élucidation de mécanismes réactionnels) ou encore le diagnostic, la recherche et le développement de nouveaux produits pharmaceutiques et thérapeutiques. Les molécules marquées $^{14}C$ présentent, en effet, un avantage pour les études métaboliques car le $^{14}C$ est facilement détectable et quantifiable en milieu *in vitro* comme *in vivo.*

La source principale de $^{14}C$ est le $^{14}CO_2$ qui est obtenu par acidification du carbonate de baryum $Ba^{14}CO_3$. La mise au point de procédés de synthèse de molécules de base servant à l'élaboration de médicaments est donc primordiale pour produire des principes actifs marqués $^{14}C$ dont le métabolisme pourra ainsi être déterminé (R. Voges, J. R. Heys, T. Moenius, "Preparation of Compounds Labeled with Tritium and Carbon-14". Wiley-VCH: Chippenham (UK), 2009).

La contrainte majeure limitant la synthèse de molécules marquées $^{14}C$ est la nécessité d'avoir un rendement élevé en produit $^{14}C$ formé par rapport à la quantité de $^{14}CO_2$ utilisée et de reposer sur un nombre restreint d'étapes afin de limiter au maximum les coûts liés à l'utilisation de $Ba^{14}CO_3$ (U. Pleiss, R. Voges, "Synthesis and Applications of Isotopically Labelled Compounds, Volume 7". Wiley-VCH, 2001 ; R. Voges, J. R. Heys, T. Moenius, "Preparation of Compounds Labeled with Tritium and Carbon-14". Wiley-VCH: Chippenham (UK), 2009).

Le procédé selon l'invention répond à ces exigences car la pression de travail en $CO_2$ peut être faible, par exemple de 0,2 à 1 bar. En outre, le taux d'incorporation en $CO_2$ (ou rendement par rapport au $CO_2$ introduit) reste élevé, et peut, par exemple, dépasser les 95%.

Les conditions de température, de durée de réaction, de solvant, ainsi que les quantités de réactifs et de catalyseurs mises en oeuvre dans le procédé de préparation d'amines méthylées marquées de formule (I') sont celles décrites précédemment dans le cadre du procédé de préparation d'amines méthylées de formule (I).

Enfin, la synthèse d'amine méthylées marquées $^{14}C$ selon la présente invention, est une amélioration très nette par rapport aux technologies connues reposant, généralement, sur un minimum de trois étapes, dans lesquelles le $CO_2$

est d'abord réduit en méthanol pour être ensuite converti en iodure de méthyle. Ce dernier est ensuite utilisé comme réactif méthylant et mis à réagir avec une amine pour former l'amine méthylée (S. C. Choudhry, L. Serico, J. Cupano, Journal of Organic Chemistry, 1989, vol. 54, p. 3755-3757).

Le procédé de l'invention peut donc permettre, d'accéder aux amines méthylées en une seule étape à partir du $CO_2$ avec de bons rendements et une bonne sélectivité. L'intérêt des amines méthylées marquées $^{14}C$ pour la synthèse de molécules complexes marquées $^{14}C$ est illustrée dans les références suivantes dans le cas de principes actifs pharmaceutiques : J. R. Ferguson, S. J. Hollis, G. A. Johnston, K. W. Lumbard, A. V. Stachulski, J Labelled Compd Rad 2002, Vol. 45, p. 107-113 ; T. Kikuchi, K. Fukushi, N. Ikota, T. Ueda, S. Nagatsuka, Y. Arano, T. Irie, J Labelled Compd Rad 2001, Vol. 44, p. 31-41 ; T. Okamura, T. Kikuchi, K. Fukushi, T. Irie, J Med Chem 2009, Vol. 52, p. 7284-7288 ; C. Trefzer, M. Rengifo-Gonzalez, M. J. Hinner, P. Schneider, V. Makarov, S. T. Cole, K. Johnsson, J Am Chem Soc 2010, Vol. 132, p. 13663-13665.

L'invention a également pour objet l'utilisation du procédé de préparation d'amines méthylées de formule (I) selon l'invention, dans la fabrication de vitamines, de produits pharmaceutiques, de colles, de fibres acryliques et cuirs synthétiques, de pesticides, et d'engrais.

L'invention a également pour objet l'utilisation du procédé de préparation d'amines méthylées de formule (I') selon l'invention, dans la fabrication de radiotraceurs et de radiomarqueurs. A titre d'exemples de radiotraceurs et de radiomarqueurs, on peut citer le 6-bromo-7-[$^{11}C$]méthylpurine et le 6-bromo-7-[$^{14}C$]méthylpurine, le [N-[$^{14}C$]methyl]-2-(4'-(methylamino) phenyl)-6-hydroxybenzothiazole (appelé également le [$^{14}C$]PIB) dont les structures sont représentées ci-dessous :

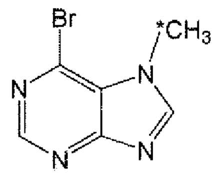

6-bromo-7-[*C]méthylpurine
*C=$^{11}C$ ou $^{14}C$

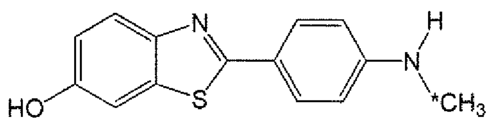

[*C]PIB
*C=$^{11}C$ ou $^{14}C$

L'invention a, en outre, pour objet un procédé de fabrication de vitamines, de produits pharmaceutiques, de colles, de fibres acryliques, de cuirs synthétiques, de pesticides, d'engrais, caractérisé en ce qu'il comprend une étape de préparation d'amines méthylées de formule (I) par le procédé selon l'invention.

L'invention a, en outre, pour objet un procédé de fabrication de traceurs et de radiotraceurs, caractérisé en ce qu'il comprend une étape de préparation d'amines méthylées de formule (I') par le procédé selon l'invention.

Comme déjà indiqué, le procédé selon l'invention conduit à la formation d'amines méthylées avec un bon rendement (de l'ordre de 35 à 100%, par exemple), et une bonne voire excellente sélectivité (par exemple, plus de 50% voire plus de 70% d'amines méthylées isolées). Une simple filtration peut permettre de récupérer le catalyseur éventuellement supporté et d'éliminer les éventuels sous-produits silylés formés.

Que ce soit pour la préparation des aminées méthylées de formule (I) ou des amines méthylées marquées de formule (I'), outre le bon rendement et la très bonne sélectivité, le procédé de l'invention permet d'obtenir lesdites amines méthylées en une seule étape, et d'utiliser

- le $CO_2$ comme source de carbone, et
- un agent réducteur doux (le silane de formule (III) ou (III')), compatible avec la présence éventuelle de groupements fonctionnels sur l'amine.

D'autres avantages et caractéristiques de la présente invention apparaîtront à la lecture des exemples ci-dessous donnés à titre illustratif et la Figure 1 annexée qui représente la stabilité thermodynamique du dioxyde de carbone.

## EXEMPLES

EXEMPLE 1 :

Le procédé de préparation d'amines méthylées de formule (I), peut être effectué selon le protocole expérimental suivant.

Les réactifs utilisés, notamment l'amine $R^1R^2NH$, le (pré-)catalyseur et le composé silane, sont des produits

commerciaux.

Sous atmosphère inerte, en boîte à gants, l'amine $R^1R^2NH$ (1 équivalent molaire), le (pré)catalyseur (de 0,001 à 1 équivalent molaire), le composé silane (1 à 3 équivalents molaires) et le solvant sont introduits dans un tube de Schlenk qui est ensuite scellé par un robinet J. Young. La concentration en amine et en composé silane dans le mélange réactionnel est d'environ 1M (concentration calculée sur la base du volume de solvant introduit). L'ordre d'introduction des réactifs n'a pas d'importance.

Le tube de Schlenk est ensuite mis sous pression de $CO_2$ (de 1 à 3 bar) à l'aide d'une rampe à vide puis est chauffé à une température comprise entre 25 et 100 °C jusqu'à la conversion totale de l'amine (de 5 minutes à 72 heures de réaction).

La réaction une fois terminée, les composés volatils sont éliminés sous pression réduite et le mélange réactionnel est purifié par chromatographie sur gel de silice. L'utilisation d'un mélange toluène/hexane comme éluant permet d'obtenir l'amine méthylée analytiquement pure.

Alternativement, si la température d'ébullition de l'amine méthylée de formule (I) est suffisamment faible (< 200°C), elle peut être isolée du mélange réactionnel par simple distillation à pression ambiante ou réduite.

Un ensemble de résultats est présenté ci-dessous, donnant des exemples de conversions d'amines primaires et secondaires en amines méthylées (déterminées par RMN) en utilisant le phénylsilane $PhSiH_3$ (commercialisé par Aldrich) et le polyméthylhydrosiloxane (PMHS) (commercialisé par Aldrich sous la référence 176206) comme réducteurs, selon les conditions testées. Les structures des amines et des (pré)catalyseurs et des silanes testés sont représentées à chaque fois.

Le schéma réactionnel est le suivant :

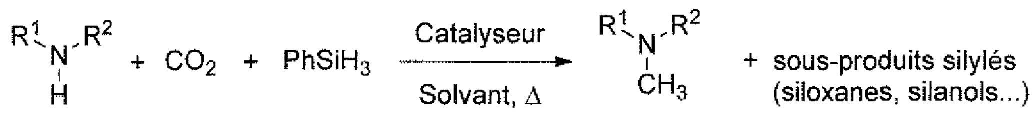


Différents (pré)catalyseurs ont été testés pour la réaction. Les résultats sont indiqués dans les tableaux suivants.

**Tableau 1 :** (Pré)catalyseurs mettant enjeu des sels ou complexes de zinc

| **Amine $R^1R^2NH$** | **Silane** | **(Pré)catalyseur** | **Conditions de réactions** | **Produit $R^1R^2NCH_3$ (a)** |
|---|---|---|---|---|
| 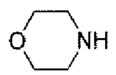 | $PhSiH_{3\ (2éq)}$ | IPr (5 mol%) + $Zn(OAc)_2$ (10 mol%) | THF<br>25°C (1 h)<br>100°C (24 h) | 65% |
| 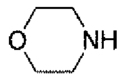 | $PhSiH_{3\ (2éq)}$ | IPr (15 mol%) + [$Zn(OAc)_2$Pyridine] (10 mol%) | THF<br>25°C (1 h)<br>100°C (24 h) | 89% |
| 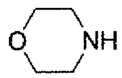 | $PhSiH_{3\ (2éq)}$ | IPr (5 mol%) + [$ZnEt_2$] (20 mol%) | THF<br>25°C (1 h)<br>100°C (24 h) | 60% |
| 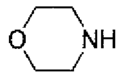 | $PhSiH_{3\ (2éq)}$ | IPr (15 mol%) + [Zn(BDI)Et] (10 mol%) | THF<br>25°C (1 h)<br>100°C (24 h) | 38% |
| 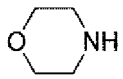 | $PhSiH_{3\ (2éq)}$ | IPr (5 mol%) + $ZnEt_2$ (10 mol%) | THF<br>25°C (1 h)<br>100°C (72 h) | 90% |
| 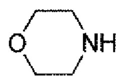 | $PhSiH_{3\ (2éq)}$ | IPr (5 mol%) + $ZnEt_2$ (10 mol%) | THF<br>100°C (72 h) | 65% |
| 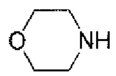 | $PhSiH_{3\ (2éq)}$ | $ZnEt_2$ (10 mol%) | THF<br>100°C (72 h) | 65% |

(suite)

| Amine $R^1R^2NH$ | Silane | (Pré)catalyseur | Conditions de réactions | Produit $R^1R^2NCH_3$ [(a)] |
|---|---|---|---|---|
| 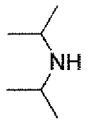 | $PhSiH_{3\ (2éq)}$ | $ZnEt_2$ (5 mol%) | THF 100°C (90 h) | 35% |
| 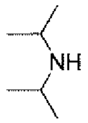 | $PhSiH_{3\ (2éq)}$ | IPr (5 mol%) + $ZnCl_2$ (5 mol%) | THF 100°C (20 h) | 46% |
| 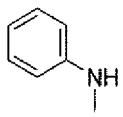 | $PhSiH_{3\ (2éq)}$ | $ZnEt_2$ (5 mol%) | THF 100°C (90 h) | 40% |
| $Ph-CH_2-NH_2$ | $PhSiH_{3\ (2éq)}$ | IPr (5 mol%) + $ZnCl_2$ (5 mol%) | THF 100°C (20 h) | 20% |
| $Ph-NH_2$ | $PhSiH_{3\ (2éq)}$ | IPr (5 mol%) + $ZnCl_2$ (5 mol%) | THF 100°C (20 h) | 62% |
| 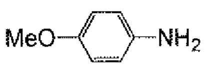 | $PhSiH_{3\ (2éq)}$ | IPr (5 mol%) + $ZnCl_2$ (5 mol%) | THF 100°C (20 h) | 67% |
| 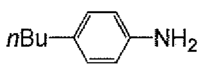 | $PhSiH_{3\ (2éq)}$ | IPr (5 mol%) + $ZnCl_2$ (5 mol%) | THF 100°C (20 h) | 49% |
| 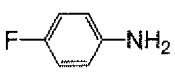 | $PhSiH_{3\ (2éq)}$ | IPr (5 mol%) + $ZnCl_2$ (5 mol%) | THF 100°C (20 h) | 67% |
| 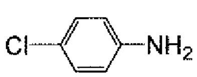 | $PhSiH_{3\ (2éq)}$ | IPr (5 mol%) + $ZnCl_2$ (5 mol%) | THF 100°C (20 h) | 49% |
| 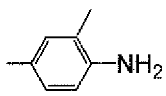 | $PhSiH_{3\ (2éq)}$ | IPr (5 mol%) + $ZnCl_2$ (5 mol%) | THF 100°C (20 h) | 12% |
| 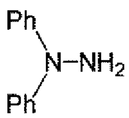 | $phSiH_3(2éq)$ | IPr (5 mol%) + $ZnCl_2$ (5 mol%) | THF 100°C (20 h) | 10% |
| $Ph_2N-NH_2$ | $PhSiH_{3\ (2éq)}$ | IPr (5 mol%) + $ZnCl_2$ (5 mol%) | THF 100°C (20 h) | 24% |

Il est à noter que « IPr » correspond au Carbène A. [(a)]Les rendements en amines méthylées $R^1R^2NCH_3$ n'ont pas été optimisés. Ainsi, les rendements modestes mais encourageants obtenus pour certaines amines restent à optimiser.

Les résultats montrent que, dans les conditions opératoires indiquées dans le tableau 1, tous les (pré)catalyseurs de zinc sont d'excellents (pré)catalyseurs car ils permettent d'obtenir les amines méthylées avec un bon voire un très bon rendement (allant de 35 à 90%°).

**Tableau 2** : (Pré)catalyseurs mettant en jeu des sels ou complexes de fer

| Amine $R^1R^2NH$ | Silane | (Pré)catalyseur | Conditions de réactions | Produit $R^1R^2NCH_3$ |
|---|---|---|---|---|
| 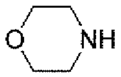 | $PhSiH_{3\ (2éq)}$ | $Fe(BF_4)_2(H_2O)_6$ + $PP_3$ (5 mol%) | THF<br>100°C (24 h) | 20% |
| 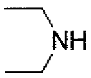 | $PhSiH_{3\ (1éq)}$ | $Fe(BF_4)_2(H_2O)_6$ + $PP_3$ (5 mol%) | THF<br>100°C (24 h) | 5% |
| 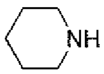 | $PhSiH_{3\ (1éq)}$ | $Fe(BF_4)_2(H_2O)_6$ + $PP_3$ (5 mol%) | THF<br>100°C (24 h) | 15% |
| 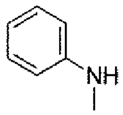 | $PhSiH_{3\ (1éq)}$ | $Fe(BF_4)_2(H_2O)_6$ + $PP_3$ (5 mol%) | THF<br>100°C (24 h) | 25% |
| 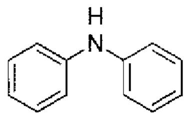 | $PhSiH_{3\ (1éq)}$ | $Fe(BF_4)_2(H_2O)_6$ + $PP_3$ (5 mol%) | THF<br>100°C (24 h) | 20% |
| 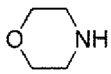 | $PhSiH_{3\ (2éq)}$ | $Fe(BF_4)_2(H_2O)_6$ + $PP_3$ (5 mol%) | THF<br>100°C (70 h) | 35% |
| 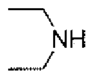 | $PhSiH_3$ (1éq) | $Fe(acac)_3$ (3,5 mol%) + $PP_3$ (5 mol%) | THF<br>100°C (24 h) | 25% |
| 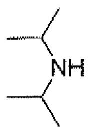 | $PhSiH_{3\ (1éq)}$ | $Fe(acac)_3$ (3,5 mol%) + $PP_3$ (5 mol%) | THF<br>100°C (24 h) | 45% |
| 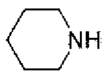 | $PhSiH_{3\ (1éq)}$ | $Fe(acac)_3$ (3,5 mol%) + $PP_3$ (5 mol%) | THF<br>100°C (24 h) | 5% |

Les résultats montrent que, dans les conditions opératoires indiquées dans le tableau 2, les (pré)catalyseurs les plus actifs résultent des mélanges $Fe(acac)_3$ + $PP_3$ et $Fe(BF_4)_2(H_2O)_6$ + $PP_3$. Pour les autres (pré)catalyseurs, une optimisation des conditions opératoires peut être envisagée.

**Tableau 3** : (Pré)catalyseurs mettant en jeu une molécule organique

| Amine $R^1R^2NH$ | Silane | (Pré)catalyseur | Conditions de réactions | Produit $R^1R^2NCH_3$ |
|---|---|---|---|---|
| 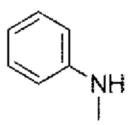 | $PhSiH_{3\ (1éq)}$ | IPr (5 mol%) | THF<br>70°C (24 h) | 50% |

Dans les conditions opératoires indiquées dans le tableau 3, l'amine méthylée est obtenue avec un bon rendement.

**Tableau 4** : (Pré)catalyseurs mettant enjeu des sels ou complexes de cuivre

| Amine $R^1R^2NH$ | Silane | (Pré)catalyseur | Conditions de réactions | Produit $R^1R^2NCH_3$ |
|---|---|---|---|---|
| 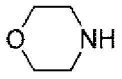 | PMHS $_{(3éq)}$ | $Cu(OAc)_2(H_2O)$ (5 mol%) dppb (7,5 mol%) | THF<br>100°C (72 h) | 40% |
| 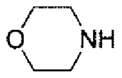 | $PhSiH_3$ $_{(2éq)}$ | $Cu(OAc)_2(H_2O)$ (5 mol%) dppb (7,5 mol%) | THF<br>100°C (72 h) | 20% |
| 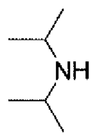 | PMHS $_{(3éq)}$ | $Cu(OAc)_2(H_2O)$ (5 mol%) dppb (7,5 mol%) | THF<br>100°C (72 h) | 25% |
| 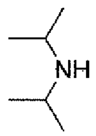 | $PhSiH_3$ $_{(2éq)}$ | $Cu(OAc)_2(H_2O)$ (5 mol%) dppb (7,5 mol%) | THF<br>100°C (72 h) | 20% |

Les résultats montrent que, dans les conditions opératoires indiquées dans le tableau 4, le phénylsilane $PhSiH_3$ peut être remplacé par un polysilane (silane de structure polymérique), le polyméthylhydrosiloxane (PMHS). Dans le cas du PMHS, le nombre d'équivalent molaire de silane s'entend comme le nombre d'équivalent molaire en unité monomérique (MeSiHO).

Les abréviations utilisées sont représentées ci-dessous :

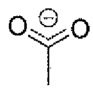
ligand OAc

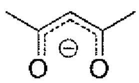
ligand acac

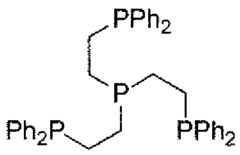
$PP_3$

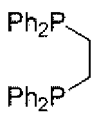
dppe

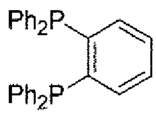
dppb

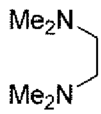
TMEDA

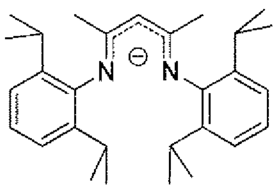
ligand BDI

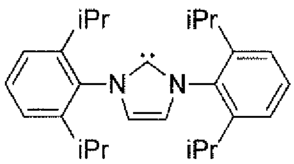
IPr

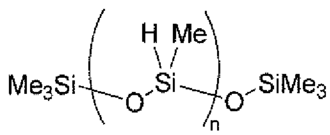
PMHS

Ces résultats montrent que la préparation des amines méthylées par le procédé de l'invention est suffisamment flexible pour convertir efficacement une grande variété d'amines secondaires, aliphatiques, aromatiques et hétérocycliques, et ce aussi bien avec des sels et complexes métalliques qu'avec des molécules organiques comme (pré)catalyseur, dans des conditions de pressions de $CO_2$ et de températures de réaction douces.

**Revendications**

1. Procédé de préparation d'amines méthylées de formule (I) :

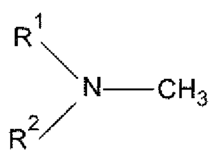
(I)

dans laquelle

- $R^1$ et $R^2$, représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, un groupe amino, une aldimine de formule $-N=CHR^6$, une cétimine de formule $-N=CR^6R^7$, lesdits groupes alkyle, alcényle, alcynyle, aryle, hétéroaryle, hétérocycle, silylé, siloxy et amino étant éventuellement substitués, ou
- $R^1$ et $R^2$, pris ensemble avec l'atome d'azote auquel ils sont liés forment un hétérocycle éventuellement substitué, ou
- $R^1$ et $R^2$, forment avec l'atome d'azote auquel ils sont liés, une double liaison carbone-azote (N=C) pour conduire à une aldimine de formule $-N=CHR^6$ ou à une cétimine de formule $-N=CR^6R^7$,
et
- $R^6$ et $R^7$, représentant, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, un groupe amino, lesdits groupes alkyle, alcényle, alcynyle, aryle, hétéroaryle, hétérocycle, silylé, siloxy et amino étant éventuellement substitués,
- $R^1$, $R^2$, $R^6$ et $R^7$ comportent éventuellement, un H, C, N, O, F, Si et/ou S tels que définis ci-dessous ;
- H représente un atome d'hydrogène ($^1H$), le deutérium ($^2H$) ou le tritium ($^3H$) ;
- C représente un atome de carbone ($^{12}C$), un isotope $^{11}C$, $^{13}C$ ou $^{14}C$ ;
- N représente un atome d'azote ($^{14}N$), un isotope $^{15}N$ ;
- O représente un atome d'azote ($^{16}O$), un isotope $^{18}O$ ;
- F représente un atome d'azote ($^{19}F$), un isotope $^{18}F$ ;
- Si représente un atome de silicium ($^{28}Si$), un isotope $^{29}Si$ ou $^{30}Si$ ;
- S représente un atome de soufre ($^{32}S$), un isotope $^{33}S$, $^{34}S$ ou $^{36}S$ ;

**caractérisé en ce que** l'on fait réagir une amine de formule (II) dans laquelle $R^1$ et $R^2$ et N sont tels que définis ci-dessus,

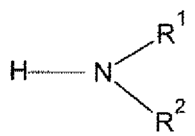

(II)

avec du $CO_2$ dans lequel C et O sont tels que définis ci-dessus ; en présence d'un catalyseur et d'un composé silane de formule (III)

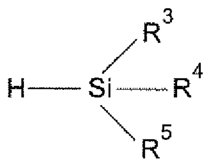

(III)

dans laquelle

- H est tel que défini ci-dessus,
- $R^3$, $R^4$ et $R^5$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^5$ est tel que défini ci-dessus et $R^3$ et $R^4$, pris ensemble avec l'atome de silicium auquel ils sont liés forment un hétérocycle silylé éventuellement substitué.

2. Procédé selon la revendication 1, **caractérisé en ce que** dans l'amine de formule (II), $R^1$ et $R^2$, représentent, indépendamment l'un de l'autre, un atome d'hydrogène, un groupe alkyle, un groupe aryle, un groupe hétéroaryle, lesdits groupes alkyle, aryle et hétéroaryle étant éventuellement substitués, ou
$R^1$ et $R^2$, pris ensemble avec l'atome d'azote auquel ils sont liés forment un hétérocycle éventuellement substitué.

**3.** Procédé selon l'une des revendications 1 ou 2, **caractérisé en ce que** dans le composé silane de formule (III), $R^3$, $R^4$ et $R^5$ représentent, indépendamment l'un de l'autre, un atome d'hydrogène, un groupe alkyle, un groupe alkoxy, un groupe aryle, un groupe silylé, un groupe siloxy, lesdits groupes alkyle, alkoxy, silylé, siloxy et aryle étant éventuellement substitués.

**4.** Procédé selon l'une quelconque des revendications 1 à 3, **caractérisé en ce que** le catalyseur est choisi parmi les catalyseurs organiques ou les catalyseurs métalliques, les catalyseurs métalliques étant choisis parmi les sels ou les complexes métalliques.

**5.** Procédé selon la revendication 4, **caractérisé en ce que** le catalyseur organique est :

- une amine secondaire ou tertiaire choisie parmi le triazabicyclodécène (TBD) ; le N-méthyltriazabicyclodécène (MeTBD), le 1,8-diazabicyclo[5.4.0]undéc-7-éne (DBU), la triméthylamine, la triéthylemaine, la pipéridine, la 4-diméthylaminopyridine (DMAP), le 1,4-diazabicyclo[2.2.2]octane (DABCO), la proline, le phénylalanine, un sel de thiazolium, la *N*-diisopropyléthylamine (DIPEA ou DIEA) ; ou
- un carbène N-hétérocyclique comme un carbène issu d'un sel d'imidazolium choisi parmi les sels de 1,3-his(2,6-diisopropylphenyl)-1H-imidazol-3-ium (carbène A), 1,3-bis(2,6-diisopropylphenyl)-4,5-dihydro-1H-imidazol-3-ium (carbène C), 1,3-bis(2,4,6-triméthylphenyl)-1H-imidazol-3-ium (carbène B), 1,3-bis(2,4,6-triméthylphenyl)-4,5-dihydro-1H-imidazol-3-ium (carbène D), 4,5-dichloro-1,3-bis(2,6-diisopropylphenyl)-1H-imidazol-3-ium (carbène E), 1,3-di-tert-butyl-1H-imidazol-3-ium (carbène F), 1,3-di-tert-butyl-4,5-dihydro-1H-imidazol-3-ium, lesdits sels étant sous la forme de sels de chlorure.

**6.** Procédé selon la revendication 4, **caractérisé en ce que** le catalyseur métallique est choisi parmi les sels ou complexes de métaux de :

- métaux choisis parmi le bore, le silicium, l'aluminium, le gallium, l'étain, l'indium ;
- métaux alcalins choisis parmi le sodium, le potassium ;
- métaux alcalinoterreux choisis le magnésium, le calcium ;
- métaux de transition choisis parmi le nickel, le fer, le cobalt, le zinc, le cuivre, le rhodium, le ruthénium, le platine, le palladium, l'iridium ;
- terres rares choisis parmi le lanthane, le cérium, le praséodyme, le néodyme.

**7.** Procédé selon l'une quelconque des revendications 1 à 6, **caractérisé en ce que** la réaction est effectuée sous une pression de $CO_2$, comprise entre 1 et 50 bars, bornes incluses.

**8.** Procédé selon l'une quelconque des revendications 1 à 7, **caractérisé en ce que** la réaction est effectuée à une température comprise entre 25 et 150°C, bornes incluses.

**9.** Procédé selon l'une quelconque des revendications 1 à 8, **caractérisé en ce que** la réaction est effectuée pendant une durée de 5 minutes à 72 heures, bornes incluses.

**10.** Procédé selon l'une quelconque des revendications 1 à 9, **caractérisé en ce que** la réaction est effectuée dans un ou un mélange d'au moins deux solvant(s) choisi(s) parmi :

- les éthers, de préférence, l'éther diéthylique, ou le THF ;
- les hydrocarbures, de préférence, le benzène, ou le toluène ;
- les solvants azotés, de préférence, la pyridine, ou l'acétonitrile ;
- les sulfoxydes, de préférence, le diméthylesulfoxyde ;
- les halogénures d'alkyle, de préférence, le chloroforme, ou le chlorure de méthylène.

**11.** Procédé selon l'une quelconque des revendications 1 à 9, **caractérisé en ce que** le solvant est l'amine de formule (II).

**12.** Procédé selon l'une quelconque des revendications 1 à 11, **caractérisé en ce que** le rapport molaire entre le composé silane de formule (III) et l'amine de formule (II) est de 1 à 10, bornes incluses.

**13.** Procédé selon l'une quelconque des revendications 1 à 12, **caractérisé en ce que** la quantité de catalyseur est de 0,001 à 1 équivalent molaire, bornes incluses, par rapport à l'amine de formule (II).

**14.** Utilisation d'un procédé de préparation d'amines méthylées de formule (I) selon l'une quelconque des revendications 1 à 13, dans la fabrication de vitamines, de produits pharmaceutiques, de colles, de fibres acryliques, de cuirs synthétiques, de pesticides, et d'engrais.

**15.** Procédé de fabrication de traceurs et de radiotraceurs, **caractérisé en ce qu'**il comprend une étape de préparation d'amines méthylées marquées de formule (I) selon l'une quelconque des revendications 1 à 13.

**Patentansprüche**

**1.** Verfahren zur Herstellung von methylierten Aminen mit der folgenden Formel (I):

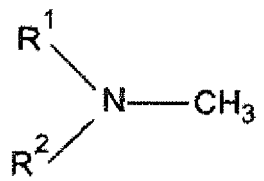

(I)

wobei

$R^1$ und $R^2$ unabhängig voneinander ein Wasserstoffatom, eine Alkylgruppe, eine Alkenylgruppe, eine Alkinylgruppe, eine Arylgruppe, eine Heteroarylgruppe, einen Heterocyclus, eine Silylgruppe, eine Siloxygruppe, eine Aminogruppe, ein Aldimin mit der Formel $-N=CHR^6$, ein Cetimin mit der Formel $-N=CR^6R^7$ darstellen, wobei die Alkyl-, Alkenyl-, Alkinyl-, Aryl-, Heteroaryl-, Heterocyclus-, Silyl, Siloxy- und Aminogruppen eventuell substituiert sind, oder

- $R^1$ und $R^2$, zusammen mit dem Stickstoffatom, an das sie gebunden sind, einen Heterocyclus bilden, der eventuell substituiert ist, oder
- $R^1$ und $R^2$ mit dem Stickstoffatom, an das sie gebunden sind, eine Doppelbindung Kohlenstoff-Stickstoff (N=C) bilden, um zu einem Aldimin mit der Formel $-N=CHR^6$·oder einem Cetimin mit der Formel $-N=CR^6R^7$ zu führen,

und

- $R^6$ und $R^7$ unabhängig voneinander ein Wasserstoffatom, eine Alkylgruppe, eine Alkenylgruppe, eine Alkinylgruppe, eine Arylgruppe, eine Heteroarylgruppe, einen Heterocyclus, eine Silylgruppe, eine Siloxygruppe, eine Aminogruppe darstellen, wobei die Alkyl-, Alkenyl-, Alkinyl-, Aryl-, Heteroaryl-, Heterocyclus-, Silyl-, Siloxy- und Aminogruppen eventuell substituiert sind, oder
- $R^1$ $R^2$, $R^6$ und $R^7$ eventuell ein H, C, N, O, F, Si und/oder S umfassen, wie unten definiert;
- H ein Wasserstoffatom ($^1H$), Deuterium ($^2H$) oder Tritium ($^3H$) darstellt;
- C ein Kohlenstoffatom ($^{12}C$), ein Isotop $^{11}C$, $^{13}C$ oder $^{14}C$ darstellt;
- N ein Stickstoffatom ($^{14}N$), ein Isotop $^{15}N$ darstellt;
- O ein Stickstoffatom ($^{16}O$), ein Isotop $^{18}O$ darstellt;
- F ein Stickstoffatom ($^{19}F$), ein Isotop $^{18}F$ darstellt;
- Si ein Siliciumatom ($^{28}Si$), ein Isotop $^{29}Si$ oder $^{30}Si$ darstellt;
- S ein Schwefelatom ($^{32}S$), ein Isotop $^{33}S$, $^{34}S$ oder $^{36}S$ darstellt;

**dadurch gekennzeichnet, dass** ein Amin mit der Formel (II) reagiert wird, wobei $R^1$ und $R^2$ und N wie oben definiert sind,

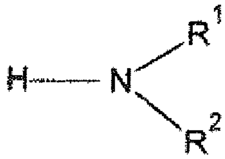

(II)

mit $CO_2$, wobei C und O wie oben definiert sind; in Anwesenheit eines Katalysators und einer Silanverbindung mit der Formel (III)

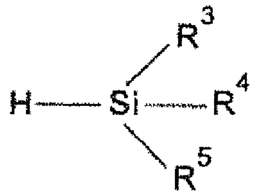

(III)

wobei

- H wie oben definiert ist,
- $R^3$, $R^4$ und $R^5$ unabhängig voneinander ein Wasserstoffatom, ein Halogenatom, eine Hydroxygruppe, eine Alkylgruppe, eine Alkenylgruppe, eine Alkinylgruppe, eine Alkoxygruppe, eine Silylgruppe, eine Siloxygruppe, eine Arylgruppe, eine Aminogruppe darstellen, wobei die Alkyl-, Alkenyl-, Alkinyl-, Alkoxy-, Silyl-, Siloxy-, Aryl- und Aminogruppen eventuell substituiert sind, oder
- $R^5$ wie oben definiert ist, und $R^3$ und $R^4$, zusammen mit dem Siliciumatom, an das sie gebunden sind, einen Silylheterocyclus bilden, der eventuell substituiert ist.

**2.** Verfahren nach Anspruch 1, **dadurch gekennzeichnet, dass** im Amin mit der Formel (II) $R^1$ und $R^2$ unabhängig voneinander ein Wasserstoffatom, eine Alkylgruppe, eine Arylgruppe, eine Heteroarylgruppe darstellen, wobei die Alkyl-, Aryl- und Heteroarylgruppen eventuell substituiert sind, oder
$R^1$ und $R^2$, zusammen mit dem Stickstoffatom, an das sie gebunden sind, einen Heterocyclus bilden, der eventuell substituiert ist.

**3.** Verfahren nach einem der Ansprüche 1 oder 2, **dadurch gekennzeichnet, dass** in der Silanverbindung mit der Formel (III) $R^3$, $R^4$ und $R^5$ unabhängig voneinander ein Wasserstoffatom, eine Alkylgruppe, eine Alkoxygruppe, eine Arylgruppe, eine Silylgruppe, eine Siloxygruppe darstellen, wobei die Alkyl-, Alkoxy-, Silyl-, Siloxy- und Arylgruppen eventuell substituiert sind.

**4.** Verfahren nach einem beliebigen der Ansprüche 1 bis 3, **dadurch gekennzeichnet, dass** der Katalysator ausgewählt ist aus den organischen Katalysatoren oder den metallischen Katalysatoren, wobei die metallischen Katalysatoren ausgewählt sind aus den Salzen oder den metallischen Komplexen.

**5.** Verfahren nach Anspruch 4, **dadurch gekennzeichnet, dass** der organische Katalysator Folgendes ist:

- ein sekundäres oder tertiäres Amin, ausgewählt aus Triazabicyclodecen (TBD); N-Methyltriazabicyclodecen (MeTBD), 1,8-Diazabicyclo[5.4.0]undec-7-en (DBU), Trimethylamin, Triethylamin, Piperidin, 4-Dimethylaminopyridin (DMAP), 1,4-Diazabicyclo[2.2.2]octan (DABCO), Prolin, Phenylalanin, einem Thiazolsalz, N-Diisopropylethylamin (DIPEA oder DIEA); oder
- einem N-heterocyclischen Carben wie einem Carben, stammend aus einem Imidazolsalz, ausgewählt aus den Salzen von 1,3-bis(2,6-Diisopropylphenyl)-1H-imidazol-3-ium (Carben A), 1,3-bis(2,6-Diisopropylphenyl)-4,5-dihydro-1H-imidazol-3-ium (Carben C), 1,3-bis(2,4,6-Trimethylphenyl)- 1 H-imidazol-3 -ium (Carben B), 1,3 - bis(2,4,6-Trimethylphenyl)-4,5-dihydro- 1 H-imidazol-3 -ium (Carben D), 4,5- Dichloro-1,3 -bis(2,6-diisopropylphenyl)- 1H-imidazol-3 -ium (Carben E), 1,3-Di-tert-butyl-1H-imidazol-3-ium (Cargen F), 1,3-di-tert-Butyl-4,5-dihydro-1H-imidazol-3-ium, wobei die Salze die Form von Chloridsalzen aufweisen.

**6.** Verfahren nach Anspruch 4, **dadurch gekennzeichnet, dass** der metallische Katalysator ausgewählt ist aus den Salzen oder den Komplexen von:

- Metallen, ausgewählt aus Bor, Silicium, Aluminium, Gallium, Zinn und Indium;
- Alkalimetalle, ausgewählt aus Natrium, Kalium;
- Erdalkalimetallen, ausgewählt aus Magnesium, Calcium;
- Übergangsmetallen, ausgewählt aus Nickel, Eisen, Kobalt, Zink, Kupfer, Rhodium, Ruthenium, Platin, Palladium, Iridium;
- Seltenerden, ausgewählt aus Lanthan, Cer, Praseodym, Neodym.

7. Verfahren nach einem beliebigen der Ansprüche 1 bis 6, **dadurch gekennzeichnet, dass** die Reaktion unter einem $CO_2$-Druck erfolgt, der zwischen 1 und 50 Bar liegt, Grenzen eingeschlossen.

8. Verfahren nach einem beliebigen der Ansprüche 1 bis 7, **dadurch gekennzeichnet, dass** die Reaktion bei einer Temperatur erfolgt, die zwischen 25 und 150 °C liegt, Grenzen eingeschlossen.

9. Verfahren nach einem beliebigen der Ansprüche 1 bis 8, **dadurch gekennzeichnet, dass** die Reaktion während einer Dauer von 5 Minuten bis 72 Stunden durchgeführt wird, Grenzen eingeschlossen.

10. Verfahren nach einem beliebigen der Ansprüche 1 bis 9, **dadurch gekennzeichnet, dass** die Reaktion in einem oder einer Mischung von mindestens zwei Lösemittel(n) durchgeführt wird, ausgewählt aus:

- Ethern, vorzugsweise Diethylether, oder THF;
- Kohlenwasserstoffen, vorzugsweise Benzen oder Toluen;
- stickstoffhaltigen Lösemitteln, vorzugsweise Pyridin oder Azetonitril;
- Sulfoxiden, vorzugsweise Dimethylsulfoxid;
- Alkylhalogenid, vorzugsweise Chloroform oder Methylenchlorid.

11. Verfahren nach einem beliebigen der Ansprüche 1 bis 9, **dadurch gekennzeichnet, dass** das Lösemittel ein Amin mit der Formel (II) ist.

12. Verfahren nach einem beliebigen der Ansprüche 1 bis 11, **dadurch gekennzeichnet, dass** das Molverhältnis zwischen der Silanverbindung mit der Formel (III) und dem Amin mit der Formel (II) von 1 bis 10 ist, Grenzen eingeschlossen.

13. Verfahren nach einem beliebigen der Ansprüche 1 bis 12, **dadurch gekennzeichnet, dass** die Menge des Katalysators von 0,001 bis 1 ist, Grenzen eingeschlossen, mit Bezug auf das Amin mit der Formel (II).

14. Verwendung eines Verfahrens zur Herstellung von methylierten Aminen mit der Formel (I) nach einem beliebigen der Ansprüche 1 bis 13 bei der Herstellung von Vitaminen, pharmazeutischen Produkten, Klebstoffen, Acrylfasern, synthetischen Ledern, Pestiziden und Dünger.

15. Verfahren zur Herstellung von Tracern und radioaktiven Tracern, **dadurch gekennzeichnet, dass** es einen Schritt des Herstellens von markierten methylierten Aminen mit der Formel (I) nach einem beliebigen der Ansprüche 1 bis 13 umfasst.

## Claims

1. Method for preparing methylated amines of formula (I):

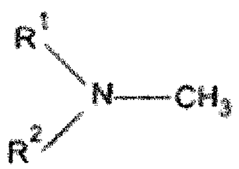

(I)

wherein

- $R^1$ and $R^2$, represent, independently from one another, a hydrogen atom, an alkyl group, an alkenyl group, an alkynyl group, an aryl group, a heteroaryl group, a heterocycle, a silyl group, a siloxy, group, an amino group, an aldimine of formula $-N=CHR^6$, a ketimine of formula $-N=CR^6R^7$, said alkyl, alkenyl, alkynyl, aryl, heteroaryl, heterocycle, silyl, siloxy and amine groups being optionally substituted, or
- $R^1$ and $R^2$, taken together with the nitrogen atom to which they are bonded form a heterocycle that is optionally substituted, or
- $R^1$ and $R^2$, form with the nitrogen atom to which they are bonded, a carbon-nitrogen double bond (N=C) in order to lead to an aldimine of formula $-N=CHR^6$ or to a ketimine of formula $_N=CR^6R^7$,

and

- $R^6$ and $R^7$, representing, independently from one another, a hydrogen atom, an alkyl group, an alkenyl group, an alkynyl group, an aryl group, a heteroaryl group, a heterocycle, a silyl group, a siloxy group, an amino group, said alkyl, alkenyl, alkynyl, aryl, heteroaryl, heterocycle, silyl, siloxy and amino groups being optionally substituted,
- $R^1$, $R^2$, $R^6$ and $R^7$ comprise optionally, an H, C, N, O, F, Si and/or S such as defined hereinbelow;
- H represents a hydrogen atom ($^1H$), deuterium ($^2H$) or tritium ($^3H$) ;
- C represents a carbon atom ($^{12}C$), an isotope $^{11}C$, $^{13}C$ or $^{14}C$;
- N represents a nitrogen atom ($^{14}N$), an isotope $^{15}N$;
- O represents a nitrogen atom ($^{16}O$), an isotope $^{18}O$ ;
- F represents a nitrogen atom ($^{19}F$), an isotope $^{18}F$;
- Si represents a silicon atom ($^{28}Si$), an isotope $^{29}Si$ or $^{30}Si$;
- S represents a sulphur atom ($^{32}S$), an isotope $^{33}S$, $^{34}S$ or $^{36}S$;

**characterised in that** an amine of formula (II) wherein $R^1$ and $R^2$ and N are such as defined hereinabove is reacted,

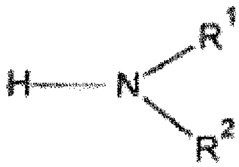

(II)

with $CO_2$ wherein C and O are such as defined hereinabove; in the presence of a catalyst and a silane compound of formula (III)

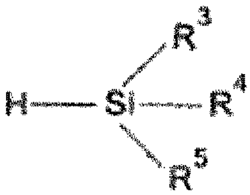

(III)

wherein

- H is such as defined hereinabove,
- $R^3$, $R^4$ and $R^5$ represent, independently from one another, a hydrogen atom, a halogen atom, a hydroxyl group, an alkyl group, an alkenyl group, an alkynyl group, an alkoxy group, a silyl group, a siloxy group, an aryl group, an amino group, said alkyl, alkenyl, alkynyl, alkoxy, silyl, siloxy, aryl and amino groups being optionally substituted, or
- $R^5$ is such as defined hereinabove and $R^3$ and $R^4$, taken together with the silicon atom to which they are bonded form a silyl heterocycle that is optionally substituted.

2. Method according to claim 1, **characterised in that** in the amine of formula (II), $R^1$ and $R^2$, represent, independently from one another, a hydrogen atom, an alkyl group, an aryl group, a heteroaryl group, said alkyl, aryl and heteroaryl groups being optionally substituted, or
$R^1$ and $R^2$, taken together with the nitrogen atom to which they are bonded form a heterocycle that is optionally substituted.

3. Method according to one of claims 1 or 2, **characterised in that** in the silane compound of formula (III), $R^3$, $R^4$ and $R^5$ represent, independently from one another, a hydrogen atom, an alkyl group, an alkoxy group, an aryl group, a silyl group, a siloxy group, said alkyl, alkoxy, silyl, siloxy and aryl groups being optionally substituted.

4. Method according to any of claims 1 to 3, **characterised in that** the catalyst is selected from organic catalysts or metal catalysts, with the metal catalysts being selected from salts or metal complexes.

**5.** Method according to claim 4, **characterised in that** the organic catalyst is:

- a secondary or tertiary amine selected from triazabicyclodecene (TBD); N-methyltriazabicyclodecene (MeTBD), 1,8-diazabicyclo[5.4.0]undec-7-ene (DBU), trimethylamine, triethylemaine, piperidine, 4-dimethylaminopyridine (DMAP), 1,4-diazabicyclo[2.2.2]octane (DABCO), proline, phenylalanine, a thiazolium salt, N-diisopropylethylamine (DIPEA or DIEA); or
- a N-heterocyclic carbene such as a carbene derived from an imidazolium salt selected from salts of 1,3-bis(2,6-diisopropylphenyl)-1H-imidazol-3-ium (carbene A), 1,3-bis(2,6-diisopropylphenyl)-4,5-dihydro-4H-imidazol-3-ium (carbene C), 1,3-bis(2,4,6-trimethylphenyl)-1H-imidazol-3-ium (carbene B), 1,3-bis(2,4,6-trimethylphenyl)-4,5-dihydro-1H-imidazol-3-ium (carbene D), 4,5-dichloro-1,3-bis(2,6-diisopropylphenyl)-1H-imidazol-3-ium (carbene E), 1,3-di-tert-butyl-1H-imidazol-3-ium (carbene F), 1,3-di-tert-butyl-4,5-dihydro-1H-imidazol-3-ium, said salts being in the form of chloride salts.

**6.** Method according to claim 4, **characterised in that** the metal catalyst is selected from salts or metal complexes of:

- metals selected from boron, silicon, aluminium, gallium, tin, indium;
- alkaline metals selected from sodium, potassium;
- alkali earth metals selected from magnesium, calcium;
- transition metals selected from nickel, iron, cobalt, zinc, copper, rhodium, ruthenium, platinum, palladium, iridium;
- rare earths selected from lanthanum, cerium, praseodymium, neodymium.

**7.** Method according to any of claims 1 to 6, **characterised in that** the reaction is carried out under a pressure of $CO_2$, between 1 and 50 bars, limits included.

**8.** Method according to any of claims 1 to 7, **characterised in that** the reaction is carried out at a temperature between 25 and 150°C, limits included.

**9.** Method according to any of claims 1 to 8, **characterised in that** the reaction is carried out for a duration of 5 minutes to 72 hours, limits included.

**10.** Method according to any of claims 1 to 9, **characterised in that** the reaction is carried out in a or in a mixture of at least two solvent (s) selected from:

- ethers, preferably, diethyl ether, or THF;
- hydrocarbons, preferably, benzene, or toluene;
- nitrogen solvents, preferably, pyridine, or acetonitrile;
- sulphoxides, preferably, dimethyl sulphoxide;
- alkyl halides, preferably, chloroform, or methylene chloride.

**11.** Method according to any of claims 1 to 9, **characterised in that** the solvent is the amine of formula (II).

**12.** Method according to any of claims 1 to 11, **characterised in that** the molar ratio between the silane compound of formula (III) and the amine of formula (II) is from 1 to 10, limits included.

**13.** Method according to any of claims 1 to 12, **characterised in that** the quantity of catalyst is from 0.001 to 1 molar equivalent, limits included, relative to the amine of formula (II).

**14.** Use of a method for preparing methylated amines of formula (I) according to any one of claims 1 to 13, in the manufacture of vitamins, pharmaceutical products, glues, acrylic fibres, synthetic leathers, pesticides and fertilisers.

**15.** Method of manufacturing tracers and radiotracers, **characterised in that** it comprises a step of preparing labelled methylated amines of formula (1) according to any of claims 1 to 13.

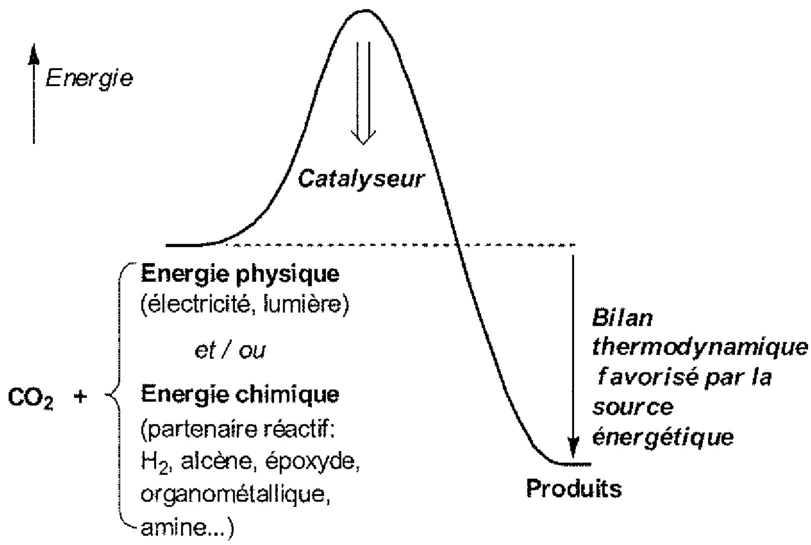
Energie
Catalyseur
Energie physique
(électricité, lumière)
et / ou
CO2 +
Energie chimique
(partenaire réactif:
H2, alcène, époxyde,
organométallique,
amine...)
Bilan
thermodynamique
favorisé par la
source
énergétique
Produits

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **MORRIS, A. J. ; MEYER, G. J. ; FUJITA, E.** *Accounts Chem Res,* 2009, vol. 42, 1983
- **RIDUAN, S. N. ; ZHANG, Y. G. ; YING, J. Y.** *Angewandte Chemie-International Edition,* 2009, vol. 48, 3322
- **SAKAKURA, T. ; CHOI, J. C. ; YASUDA, H.** *Chem Rev,* 2007, vol. 107, 2365
- **LAWRENCE, S. A.** Amines: Synthesis, Properties and Applications. Cambridge University Press, 2006
- **ARPE, H.-J. ; HAWKINS, S.** Industrial Organic Chemistry. Wiley-VCH, 1997
- **ALI, M. F. ; EL ALI, B. M. ; SPEIGHT, J. G.** Handbook of Industrial Chemistry - Organic Chemicals. McGraw-Hill, 2005
- **S. RAM ; R. E. EHRENKAUFER.** *Tetrahedron Lett.,* 1985, vol. 26 (44), 5367-5370
- **R. N. SALVATORE ; F. X. CHU ; A. S. NAGLE ; E. A. KAPXHIU ; R. M. CROSS ; K. W. JUNG.** *Tetrahedron,* 2002, vol. 58, 3329-3347
- **S. RAM ; L. D. SPICER.** *Synthetic Communications,* 1989, vol. 19, 3561-3571
- **C.L. SMITH et al.** *Journal of Organometallic Chemistry,* 1974, vol. 81, 33-40
- **G.D. HOMER.** *Journal of the American Chemical Society,* 1973, vol. 95 (23), 7700-7707
- **L. SPIALTER et al.** *Journal of the American Chemical Society,* 1971, vol. 93 (22), 5682-5686
- **R. WEST.** *Journal of the American Chemical Society,* 1954, 6015-6017
- **U. PLEISS ; R. VOGES.** Synthesis and Applications of Isotopically Labelled Compounds. Wiley-VCH, 2001, vol. 7
- **R. VOGES ; J. R. HEYS ; T. MOENIUS.** Preparation of Compounds Labeled with Tritium and Carbon-14. Wiley-VCH, 2009
- **YONG-JOO KIM ; MAX P. BERNSTEIN ; ANGELA S. GALIANO ROTH ; FLOYD E. ROMESBERG ; PAUL G. WILLIARD ; DAVID J. FULLER ; AIDAN T. HARRISON ; DAVID B. COLLUM.** *J. Org. Chem.,* 1991, vol. 56, 4435-4439
- **R. VOGES ; J. R. HEYS ; T. MOENIUS.** Préparation of Compounds Labeled with Tritium and Carbon-14. Wiley-VCH, 2009
- **T. A. KOCHINA ; D. V. VRAZHNOV ; E. N. SINOTOVA ; V. V. AVRORIN ; M. YU. KATSAP ; YU. V. MYKHOV.** *Russian Journal of General Chemistry,* 2002, vol. 72 (8), 1222-1224
- **E. A. SHISHIGIN ; V. V. AVRORIN ; T. A. KOCHINA ; E. N. SINOTOVA.** *Russian Journal of General Chemistry,* 2005, vol. 75 (1), 152
- **S. C. CHOUDHRY ; L. SERICO ; J. CUPANO.** *Journal of Organic Chemistry,* 1989, vol. 54, 3755-3757
- **J. R. FERGUSON ; S. J. HOLLIS ; G. A. JOHNSTON ; K. W. LUMBARD ; A. V. STACHULSKI.** *J Labelled Compd Rad,* 2002, vol. 45, 107-113
- **T. KIKUCHI ; K. FUKUSHI ; N. IKOTA ; T. UEDA ; S. NAGATSUKA ; Y. ARANO ; T. IRIE ; J LABELLED.** *Compd Rad,* 2001, vol. 44, 31-41
- **T. OKAMURA ; T. KIKUCHI ; K. FUKUSHI ; T. IRIE.** *J Med Chem,* 2009, vol. 52, 7284-7288
- **C. TREFZER ; M. RENGIFO-GONZALEZ ; M. J. HINNER ; P. SCHNEIDER ; V. MAKAROV ; S. T. COLE ; K. JOHNSSON.** *J Am Chem Soc,* 2010, vol. 132, 13663-13665